# EUROPEAN PATENT SPECIFICATION

(11) **EP 1 223 966 B1**
(45) Date of publication and mention of the grant of the patent: **02.05.2003**
(21) Application number: 00983097.7
(22) Date of filing: 30.10.2000
(51) Int. Cl.: A61K 38/18, A61K 48/00, A61P 27/02

(54) **USE OF GDNF FOR TREATING CORNEAL DEFECTS**
VERWENDUNG VON GDNF FÜR BEHANDLUNG VON HORNHAUTDEFEKTEN
UTILISATION DU FACTEUR GDNF DANS LE TRAITEMENT DES DEFAUTS DE LA CORNEE

(30) Priority: 29.10.1999 EP 99121597
(43) Date of publication of application: 24.07.2002
(73) Proprietor: BIOPHARM GESELLSCHAFT ZUR BIOTECHNOLOGISCHEN ENTWICKLUNG VON PHARMAKA mbH, 69115 Heidelberg (DE)
(72) Inventor: HANKE, Michael, 67454 Hassloch (DE); KRUSE, Friedrich, 68526 Ladenburg (DE); PAULISTA, Michael, 69181 Leimen (DE); POHL, Jens, 76707 Hambrücken (DE)
(74) Representative: Müller-Boré & Partner Patentanwälte
(86) International application number: EP0010674
(87) International publication number: WO01030375

(56) References cited:
- WO-A-96/14861
- WO-A-98/32458
- US-A- 5 929 041
- BATCHELOR, PETER E. ET AL: "Activated macrophages and microglia induce dopaminergic sprouting in the injured striatum and express brain-derived neurotrophic factor and glial cell line-derived neurotrophic factor" J. NEUROSCI. (1999), 19(5), 1708-1716 , XP002168455
- KRUSE F E ET AL: "Stem cells, wound healing, growth factors, and angiogenesis in the cornea." CURRENT OPINION IN OPHTHALMOLOGY, (1997 AUG) 8 (4) 46-54. REF: 72 , XP001002191
- YOU LINGTAO ET AL: "Neurotrophic factors in the human cornea." IOVS., vol. 41, no. 3, March 2000 (2000-03), pages 692-702, XP001002197

## Description

The present invention relates to the use of a glial cell line-derived growth factor (GDNF) or a functionally active derivative or part thereof and/or an agonist which substitutes the functional activity of GDNF, and/or a nucleic acid containing at least a nucleotide sequence encoding the primary amino acid sequence of GDNF or the functionally active derivative or part thereof and/or of the agonist for the manufacture of a pharmaceutical composition for epidermal and stromal wound healing.

Until now comeal wound healing disorders, in particular wound healing disorders of the comeal epithelium, are not treatable in certain patients. Such patients mostly suffer from accompanying disorders such as neurotrophic eye diseases (for example various forms of impaired nerve supply), infectious diseases (viral diseases, bacterial disease, fungal disease, chlamydial disease), localized or generalized immunological diseases (for example allergic, vernal, atopic keratokonjunctivitis), various forms of rheumatoid eye disease (for example in the context of rheumatoid arthritis, Morbus Wegener, Lupus Erythematodes, sclerodermia), Stevens-Johnson Syndrome, diseases caused by associated dermal diseases (e.g. rosazea, ichthyosis), moistening disorders (different forms of dry eye), impaired function of the lids and eye-lashes, and systemic diseases (such as diabetes, gout, M. Crohn), various forms of degenerative disease (senile, marginal, pellucid, Terrien's, Salzman's degeneration), dystrophic disease (comeal dystrophies of all three layers of the cornea including Fuchs' dystrophy) as well as various inflammations of the neighbouring tissues (conjunctiva, sclera). Further reasons for comeal wound healing disorders may be all sorts of physical injury to the ocular surface such as abrasions, cuts, lacerations due to organic and inorganic material, furthermore chemical injuries induced by solid, liquid and gaseous material as well as burns. Furthermore, wound healing disorders can be induced by medical and cosmetic intervention comprising the entire spectrum of refractive and therapeutic laser surgery (namely excimer, infrared and Nd:Yag) as well as mechanical and nonmechanical cutting in the context of refractive and conventional medical surgery (radial keratotomy, LASIK, trephination in the context of lamellar of perforating keratoplasty).

For such diseases there is currently no conservative therapy available and therefore, frequently there has to be carried out a complicated and invasive transplantation of the cornea. The same problem applies to physical injuries leading to comeal abrasions.

First studies carried out by Lambiase et al. (1998) seem to indicate that nerve growth factor (NGF) isolated from the submandibular glands of mice may be suited for the conservative therapy of neurotrophic corneal ulcer. However, murine NGF had to be used at very high doses, has to be isolated by a complicated and very expensive process and represents a nonhuman protein.

Therefore, there is a great demand for novel approaches for the conservative therapy for the healing of wounds and the treatment of wound healing disorders of epithelial and stromal tissues, in particular in the anterior eye.

Accordingly, the technical problem underlying the present invention is to provide a novel system for the healing and the treatment of healing disorders of epithelial and stromal wounds, in particular in the anterior eye.

The solution to the above technical problem is achieved by providing the embodiments as characterized in the claims.

In particular, the present invention relates to the use of a glial cell line-derived growth factor (GDNF) or a functionally active derivative or part thereof and/or an agonist which substitutes the functional activity of GDNF, and/or a nucleic acid containing at least a nucleotide sequence encoding the primary amino acid sequence of GDNF or the functionally active derivative or part thereof and/or of the agonist for the manufacture of a pharmaceutical composition for epidermal and stromal wound healing and/or for the treatment of epidermal and stromal wound healing disorders and/or scarring disorders.

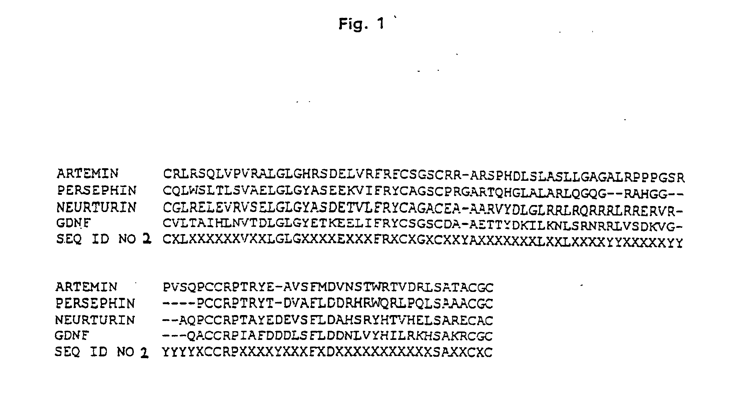

The term "glial cell line-derived growth factor (GDNF)" refers to GDNF, neurturin, persephin, artemin (also referred to as enovin or neublastin) and to all proteins capable of healing comeal defects and whose amino acid sequence comprises at least the conserved seven cyteine region and shares more than 60% identity with the amino acid sequence of the conserved seven cysteine region of human GDNF (SEQ ID NO 1). According to one embodiment of the present invention the term "GDNF" includes proteins which comprise at least the generic amino acid sequence as shown in Fig. 1 (SEQ ID NO 2) which is derived from the conserved seven cystein regions of GDNF (SEQ ID NO 1), neurturin (SEQ ID NO 3), persephin (SEQ ID NO 4) and artemin (SEQ ID NO 5), and is capable of healing corneal defects. In SEQ ID NO 2 X denotes any amino acid and Y denotes any amino acid or a deleted amino acid. The terms "functionally active derivative" and "functionally active part" refer to a proteinaceous compound exhibiting at least part of the biological function of GDNF and include polypeptides containing amino acid sequences in addition to the mature GDNF, e.g. proGDNF and preproGDNF, the mature GDNF itself as well as mutants of the wild-type GDNF polypeptide. The term "mutant" as used herein comprises polypeptides obtained by insertion, deletion and/or substitution of one or more amino acids in the wild-type GDNF primary amino acid sequence such as the human wild-type GDNF sequence. Furthermore, the term "GDNF" comprises recombinantly produced polypeptides, such as, for example, recombinant human GDNF having the amino acid sequence of human wild-type GDNF according to GenBank accession nos. L19063, L15306.

The term "agonist" as used herein means a proteinaceous or nonproteinaceous compound capable of substituting the functional activity of GDNF or the functionally active derivative or part thereof. Such agonists may exhibit a biological effect of GDNF by, for example, binding to the same receptors, such as the ret tyrosine kinase receptor and GDNF family receptor alpha 1-4 (GFRalpha1-4 ), respectively, and/or by influencing the same transductional pathways up and/or down stream of these receptors.

The functionally active form of GDNF or the agonist thereof or the functionally active derivatives or parts thereof may be a monomeric form or multihomo- or heteromultimeric form such as a dimeric, trimeric or other oligomeric form.

The term "nucleic acid" means natural or semi-synthetic or synthetic or modified nucleic acid molecules which may be composed of deoxyribonucleotides and/or ribonucleotides and/or modified nucleotides. The nucleic acid as defined above contains at least a nucleotide sequence which encodes the primary amino acid sequence of the above-defined GDNF polypeptide or the functionally active derivative such as a mutant or part thereof and/or of an above-defined proteinaceous agonist thereof. Examples of the nucleic acid according to the present invention contain a nucleotide sequence according to GenBank accession no. NM 000514 which encodes wild-type human GDNF. The nucleotide sequence according to the present invention may also be a mutant sequence resulting from insertion, deletion and/or substitution of one or more nucleotides compared to the wild-type sequence.

The pharmaceutical composition according to the present invention may also be used as a gene therapeutic or cell therapeutic agent. Therefore, according to a preferred embodiment of the present invention, the pharmaceutical composition comprises cells which are, for example, transformed by the above defined nucleic acid, which produce GDNF or the functionally active derivative or part thereof and/or the agonist thereof.

Preferably, the pharmaceutical composition as defined above contains at least one further agent having a trophic effect on epithelial and/or neuronal cells. Such agents are preferably cytokins such as TGF-βs (e.g. TGF-β1, -β2 and -β3), BMPs, GDFs, and cytokins capable of binding to TrkA, TrkB and TrkC receptors, such as neurotrophins, e.g. NGF, NT-3, NT-4/5, BDNF, CDNF) ligands of ret and GFRalpha 1-4, ligands of EGF-receptors (EGF, heparin-binding EGF-like growth factor (HB-EGF), TGF-α), various members of the fibroblast growth factor family (FGF 1-5), keratinocyte growth factor (KGF), hepatocyte growth factor (HGF), the various isoforms of platelet-derived growth factor (PDGF A, B, AB) and isoforms of insulin growth factor (IGF-I,II). Also the further agent having a trophic effect on epithelial and/or neuronal cells in combination with GDNF may be one or more components of human serum which may be used as a whole or as a part thereof, preferably in combination with fibronectin or metabolites thereof. Furthermore, GDNF may be used in combination with any kind of anti-inflammatory agents (for example steroids such as cortisone and its analogs, nonsteroidal agents such as inhibitors of the arachidonic acid pathway or the NF-κB signal transduction pathway and antibodies against chemokines). Such further agents may exhibit additive and/or synergistic effects in combination with GDNF, the agonist and/or the nucleic acid as defined above.

According to a further preferred embodiment of the use according to the present invention, the wound which is to be healed or which is prevented from normal healing due to a wound healing disorder is located in the anterior eye of a mammal. More preferably, the above defined pharmaceutical composition is used for comeal epithelial, stromal and endothelial wound healing and scarring and/or for the treatment of corneal epithelial, stromal and endothelial wound healing and scarring. An especially preferred example of a comeal wound is a corneal ulcer.

As a further example, the wound and/or wound healing disorder as defined above is caused by disorders such as neurotrophic eye diseases (for example various forms of impaired nerve supply), infectious diseases (viral diseases, bacterial disease, fungal disease, chlamydial disease), localizd or generalized immunological diseases (for example allergic, vernal, atopic keratokonjunctivitis, various forms of rheumatoid eye disease (for example in the context of rheumatoid arthritis, Morbus Wegener, Lupus Erythematodes, sclerodermia), Stevens-Johnson Syndrome, diseases caused by associated dermal diseases (e.g. rosazea, ichthyosis), moistening disorders (different forms of dry eye), impaired function of the lids and eye-lashes, and systemic diseases (such as diabetes, gout, M. Crohn), various forms of degenerative disease (senile, marginal, pellucid, Terrien's, Salzman's degeneration), dystrophic disease (corneal dystrophies of all three layers of the cornea including Fuchs' dystrophy) as well as various inflammations of the neighbouring tissues (conjunctiva, sclera). Further reasons for comeal wound healing disorders as defined above may be all sorts of physical injury to the ocular surface such as abrasions, cuts, lacerations due to organic and inorganic material, furthermore chemical injuries induced by solid, liquid and gaseous material as well as burns. Furthermore, wound healing disorders as defined above can be induced by medical and cosmetic intervention comprising the entire spectrum of refractive and therapeutic laser surgery (namely excimer, infrared and Nd:Yag) as well as mechanical and nonmechanical cutting in the context of refractive and conventional medical surgery (radial keratotomy, LASIK, trephination in the context of lamellar of perforating keratoplasty).

Preferably, the pharmaceutical composition as defined above additionally contains a pharmaceutically acceptable carrier and/or diluent and may preferably be applied orally, topically, intravenously and/or parenterally. Thereby, the carrier and/or diluent which may be used in the pharmaceutical composition according to the present invention depends on the administration route which also influences the final formulation such as, for example, ointments, eye drops, gel formulations or solutions for ocular injection.

The pharmaceutical composition according to the present invention typically includes a pharmaceutically effective amount of a GDNF or a functionally active derivative or part thereof and/or an agonist which substitutes the functional activity of GDNF, and/or the above-defined nucleic acid which encodes the primary amino acid sequence of GDNF and/or the agonist in combination with one or more pharmaceutically and physiologically acceptable formulation materials such as a carrier and/or a diluent. Further formulation components include antioxidants, preservatives, colouring, flavouring and emulsifying agents, suspending agents, solvents, fillers, bulking agents, buffers, delivery vehicles, excipients and/or pharmaceutical adjuvants. For example, a suitable carrier or vehicle may be water for injection, physiological saline solution, or a saline solution mixed with a suitable carrier protein such as serum albumin.

The solvent or diluent of the pharmaceutical composition may be either aqueous or non-aqueous and may contain other pharmaceutically acceptable excipients which are capable of modifying and/or maintaining a pH, osmolarity, viscosity, clarity, scale, sterility, stability, rate of dissolution or odour of the formulation. Similarily other components may be included in the pharmaceutical composition according to the present invention in order to modify and/or maintain the rate of release of the pharmaceutically effective substance, such as the GDNF protein product or to promote the absorption or penetration thereof across the epithelial and/or stromal cells. Such modifying components are substances usually employed in the art in order to formulate dosages for parenteral administration in either unit or multi-dose form.

The finally formulated pharmaceutical composition according to the present invention may be stored in sterile vials in form of a solution, suspension, gel, emulsion, solid or dehydrated or lyophilized powder. These formulations may be stored either in a ready-to-use form or in a form, e.g. in case of a lyophilized powder, which requires reconstitution prior to administration.

The above and further suitable pharmaceutical formulations are known in the art and are described in, for example, Gus Remington's Pharmaceutical Sciences (18th Ed., Mack Publishing Co., Eastern, PA, 1990, 1435-1712). Such formulations may influence the physical state, stability, rate of *in vivo* release and rate of *in vivo* clearance of the pharmaceutically effective component, such as the GDNF protein, the agonist and/or the nucleic acid as defined above.

Other effective administration forms comprise parenteral slow-release, i.e. retarded, formulations, inhalent mists, or orally active formulations. For example, a slow-release formulation may comprise GDNF or functionally active derivative or part thereof which may be bound to or incorporated into particulate preparations of polymeric compounds (such as polylactic acid, polyglycolic acid etc.) or lyposomes. According to a further preferred embodiment of the present invention hyaluronic acid may be used as a carrier for the pharmaceutically active component, e.g. GDNF, which may have the effect of promoting sustained duration in the circulation. The pharmaceutical composition according to the present invention may also be formulated for parenteral administration, e.g., by ocular infusion or injection, and may also include slow-release or sustained circulation formulations. Such parenterally administered therapeutic compositions are typically in the form of pyrogen-free, parenterally acceptable aqueous solutions comprising the pharmaceutically effective component(s) such as GDNF in a pharmaceutically acceptable carrier and/or diluent.

Preferred formulations of the pharmaceutical composition according to the present invention comprise typical ophthalmic preparations, including ophthalmic solutions, suspensions, ointments and gel formulations. Other administration routes are, for example, intracameral injections, which may be made directly into the interior chamber or directly into the vicious chamber of the eye, subconjunctival injections and retrobulbal injections.

Preferably, the pharmaceutical composition according to the present invention may be administered to the ocular surface and the (external) space between the eye ball and the eye lid, i.e. by extra-ocular administration. Preferred examples of extra-ocular regions include the eye lids fomix or cul-de-sac, the conjunctival surface and, more preferably, the corneal surface. This location ist external to all ocular tissue and, therefore, an invasive procedure is not required to access these regions. Preferred examples of extra-ocular administration include inserts and typically applied eye drops, gel formulations or ointments which may be used to deliver therapeutic material to the extra-ocular regions. Other possible forms of application are slow release and/or contact shields made from inorganic and/or organic material (such as biodegradable shield made, for example, of collagen), contact lenses as well as artificial and natural surface substrates such as amniotic membranes or matrices composed from various forms of collagen or artificial materials such as plastics. Such extra-ocular devices are generally easily removable even by the patient himself or herself.

Especially preferred formulations for pharmaceutical compositions for wound healing in the anterior parts of the eye, such as corneal wounds, are polymeric gel formulations comprising a polymer which may be selected from the group consisting of vinyl polymers, polyoxy ethylene-polyoxy propylene copolymers, polysaccharides, proteins, poly(ethylene oxide), acrylamide polymers and derivatives or salts thereof. Such gel formulations are described in, e.g., US patent 5,705,485. Gel formulations comprising a water-soluble, pharmaceutically or ophthalmically compatible polymeric material advantageously influence the viscosity of the pharmaceutical composition within various ranges determined by the application, since the formulations are capable of controlling the release and increased contact time of the pharmaceutically active component to the wound site. Especially preferred examples of gel formulations containing a polymeric material are hyaluronic acid gel formulations. Hyaluronic acid (HA) is one of the mucopolysaccharides having a straight chain structure consisting of the repitition of a disaccharide unit of N-acetyl glucosamine and glucuronic acid. HA is found in nature, microorganisms and in the skin in connected tissue of humans and animals. Molecular weights of HA are within the range of from 50,000 to 8,000,000 depending on source, preparation and method of determination. Viscous solutions of HA have lubricating properties and an excellent moisturizing effect. It is found in the synovial fluid of joints, vitreous body of the eye ball, umbilical cord, skin, blood vessels and cartilage. HA works remarkably well as a lubricant and shock absorbing agent, and this is probably due to its water-retaining ability and its affinity for linking of certain specific proteins. It is considered to be a very safe molecule for internal use within the human body. Thus, it may be used in the pharmaceutical composition according to the present invention for wound healing and the treatment of wound healing disorders, such as wounds in the anterior eye. Furthermore, the excellent lubricating properties and moisturizing effects of HA are highly advantageous for a pharmaceutical composition according to the present invention which may be, e.g., used for the treatment of wound healing disorders caused by different forms of dry eye. Preferably, hyaluronic acid is present in concentrations of 0.5 to 5.0 % by weight, based on the total weight of the pharmaceutical composition. Such a concentration range is suitable for the formulation of light viscous solutions which may be used as eye drops having a viscosity which is preferably in the range of 1 to 1,000 mPa^{·}s as well as for other forms of applications such as soaking bandages, wherein the viscosity is preferably in the range of 1.0 to 5,000 mPa^{·}s.

According to a preferred embodiment of the pharmaceutical composition according to the present invention, the pharmaceutically effective component, such as GDNF, preferably recombinant human GNDF, may be present in concentrations ranging from 0.01 to 1 mg/ml, for example in the case of liquid formulations such as gel formulations or formulations based on water.

The pharmaceutical composition according to the present invention may be applied, for example in the case of liquid formulations such as gel formulations or formulations based on water for topical administration, e.g. eye drops, in doses ranging from 5 to 100 µl and may be administered once to 24 times per day.

The figures show:
- Fig. 1: is a sequence alignment of the conserved seven cystein regions of GDNF (SEQ ID NO 1, GenBank accession nos. L19063, L15306), artemin (SEQ ID NO 5, GenBank accession no. AF109401), persephin (SEQ ID NO 4, GenBank accession no. AF040962), neurturin (SEQ ID NO. 3, GenBank accession no. U78110) and the resulting generic consensus sequence (SEQ ID NO. 2). In the consensus sequence of SEQ ID NO 2 X denotes any amino acid and Y denotes any or no amino acid.

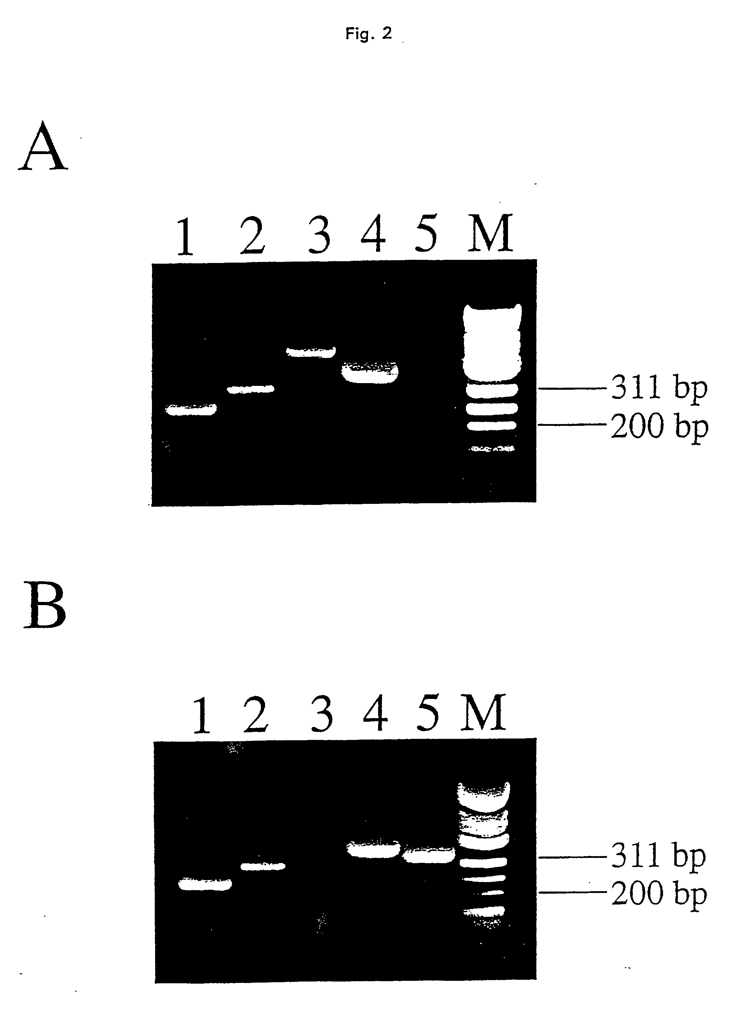

- Fig. 2: shows a 1.8% agarose gel electrophoresis of PCR products amplified from the cDNA generated from mRNA extracted from *ex vivo* comeal epithelium (A) and stroma (B) stained with ethidium bromide. Both epithelium and stroma expressed NGF (233 bp) (lane 1), NT-3 (298 bp) (lane 2), and BDNF (373 bp) (lane 4). NT-4 (464 bp) (lane 3) was only expressed in corneal epithelium. GDNF (343 bp) (lane 5) was mostly expressed in corneal stroma. M = DNA molecular weight marker (PhiX 174 DNA/Hinf I fragments).

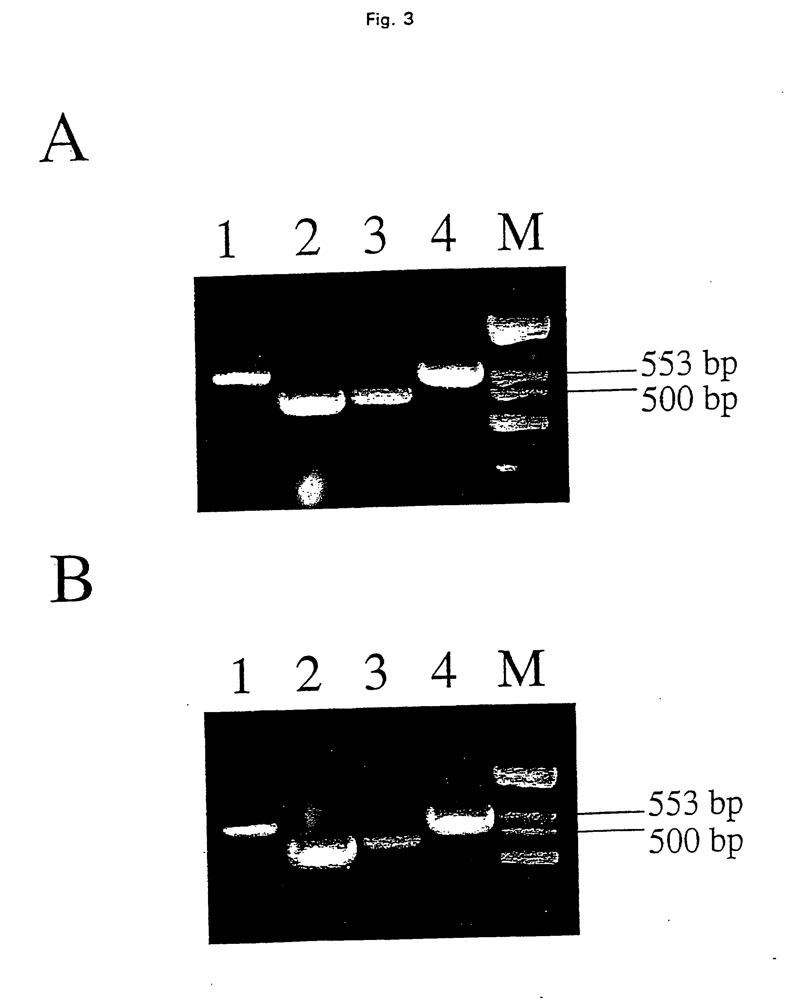

- Fig. 3: shows a 1.8% agarose gel electrophoresis of PCR products amplified from cDNA generated from mRNA extracted from ex *vivo* corneal epithelium (A) and stroma (B) stained with ethidium bromide. Both epithelium and stroma expressed the neurotrophin receptors TrkA (570 bp) (lane 1), TrkB (472 bp) (lane 2), TrkC (484 bp) (lane 3) and TrkE (545 bp) (lane 4). M = DNA molecular weight marker (PhiX 147 DNA/Hinf I fragments).

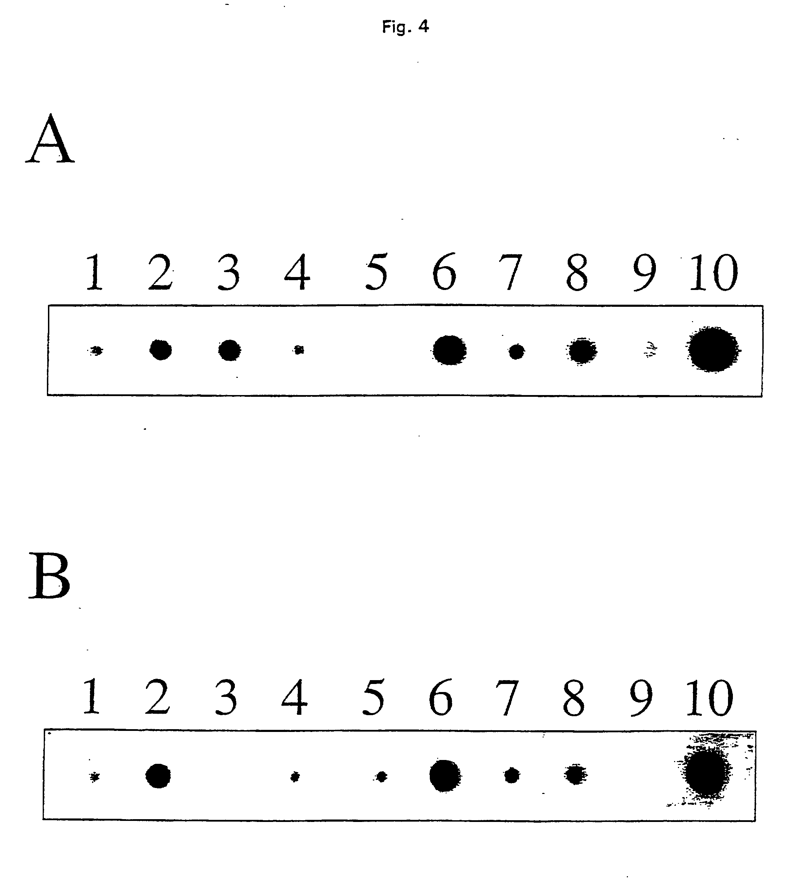

- Fig. 4: shows a DNA dot blot analysis for the determination of the transcriptional level of GDNF and other neurotrophic factors and the corresponding tyrosine kinase receptors in cultured human corneal epithelial cells (A) and stromal keratocytes (B). Each DNA dot in 1 to 10 represents 0.1 µg PCR dots specific for NGF (lane 1), NT-3 (lane 2), NT-4 (lane 3), BDNF (lane 4), GDNF (lane 5), TrkA (lane 6), TrkB (lane 7), TrkC (lane 8), TrkE (lane 9) and GAPDH (lane 10). The transcription of NT-4 was only detectable in cultured human corneal epithelial cell line and the transcription of GDNF was mostly detectable in cultured human corneal stromal keratocytes. For comparison, lane 10 represents GAPDH as positive control which shows the strongest signal.

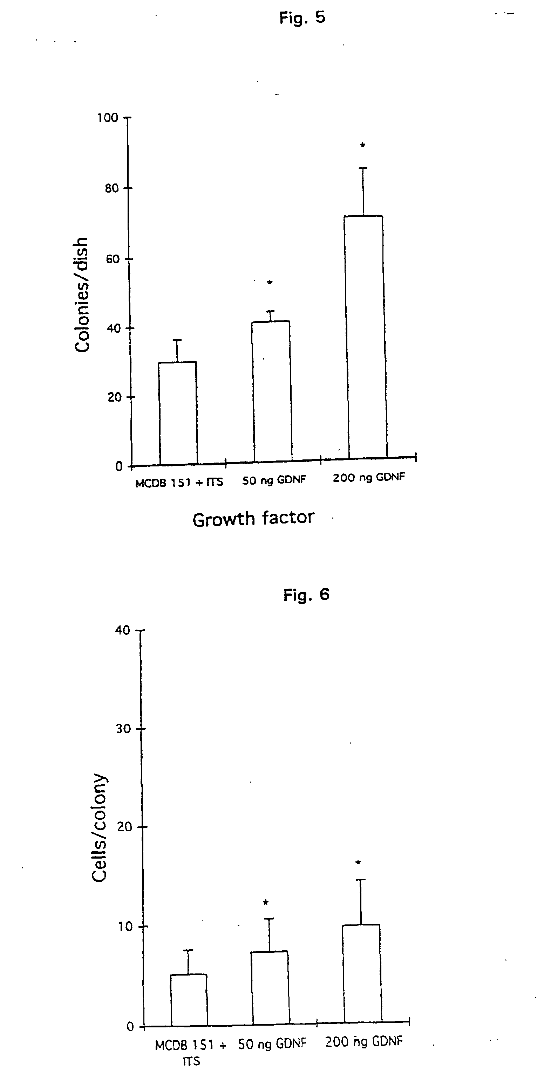

- Fig. 5: is a diagram showing the effect of recombinant human GDNF on the colony formation of primary rabbit corneal epithelial cells on D6 (mean values and standard deviations). The cells were cultured in a clonal density in serum-free MCDB. Addition of 50 or 200 ng/ml GDNF resulted in a statistically significant (p < 0.005, *) increase of the total number of colonies.
- Fig. 6: shows a diagram demonstrating the effect of recombinant human GDNF on the clonal proliferation of primary rabbit corneal epithelial cells on D6 (mean values and standard deviations). Addition of GDNF (50 or 200 ng/ml) resulted in a statistically significant (p < 0.005, *) increase of the number of cells per colony.

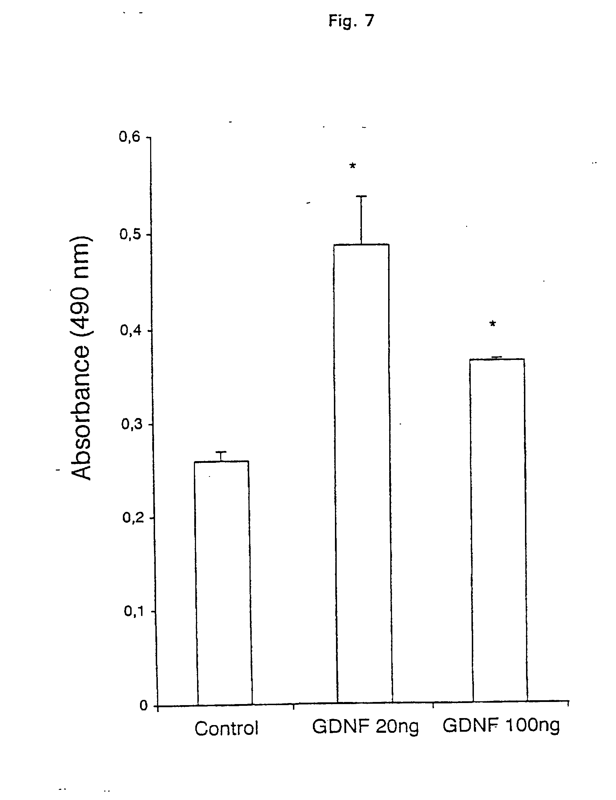

- Fig. 7: is a diagram showing the effect of GDNF on the proliferation of primary human corneal stromal cells on D6. Following culture in DMEM plus 10% FBS, cells were plated at a low density in DMEM without FBS and further processed. Values are shown as mean +/- standard deviation (SD). GDNF led to a statistically significant induction of absorbance which reflects the cell density as compared to the control (p < 0.005, *).

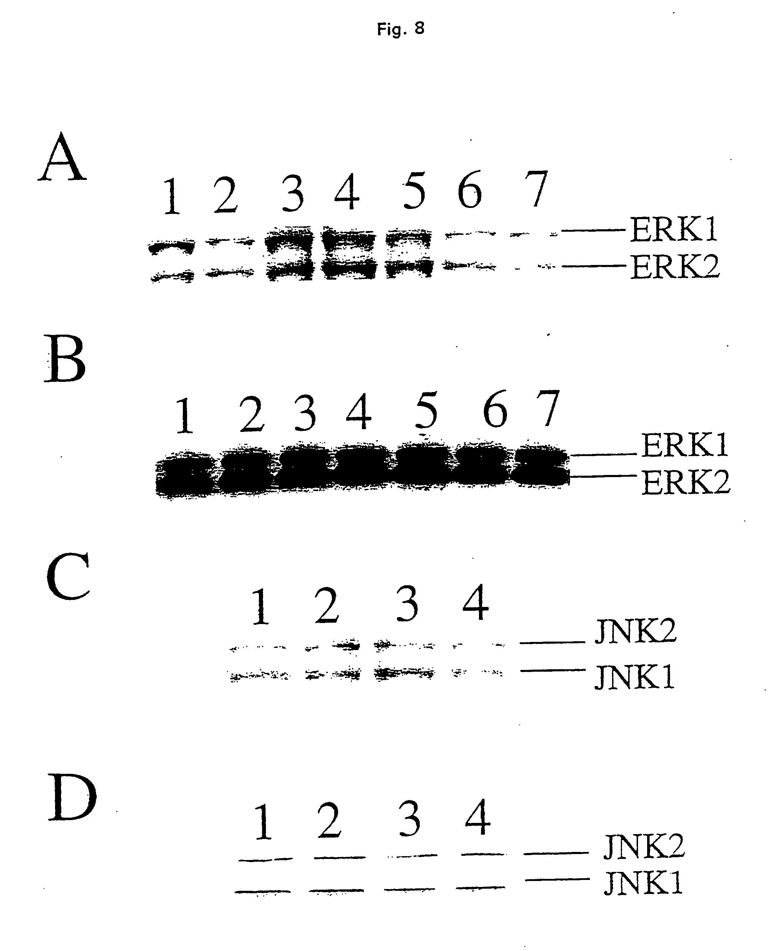

- Fig. 8: shows western blots demonstrating the effect of GDNF and other neurotrophic factors on the phosphorylation of MAP kinase in cultured human corneal epithelium. Western blots with antibodies against phosphorylated ERK1 (44 kD) and ERK2 (42 kD) (A), total ERK1 and ERK2 (phosphorylated and nonphosphorylated) (44 kD and 42 kD) (B), phosphorylated JNK1 (46 kD) and JNK2 (54 kD) (C), and against total JNK1 and JNK2 (phosphorylated and nonphosphorylated) (46 kD and 54 kD) (D) demonstrate that phosphorylation of ERK1 and to a lesser extent of ERK2 was induced in human epithelial cells cultured in medium containing GDNF (lane 5), BDNF (lane 3) or NGF (lane 1) in comparison to cells cultured in serum-free control medium (lane 7). Phosphorylation of ERK1 by NGF and GDNF but not by BDNF was inhibited by addition of the MEK-inhibitor PD 98059 (lanes 2, 6 and 4, respectively). in contrast, JNK1/2 were not induced by NGF (lane 1), BDNF (lane 2) or GDNF (lane 3) as compared to the control (lane 4).

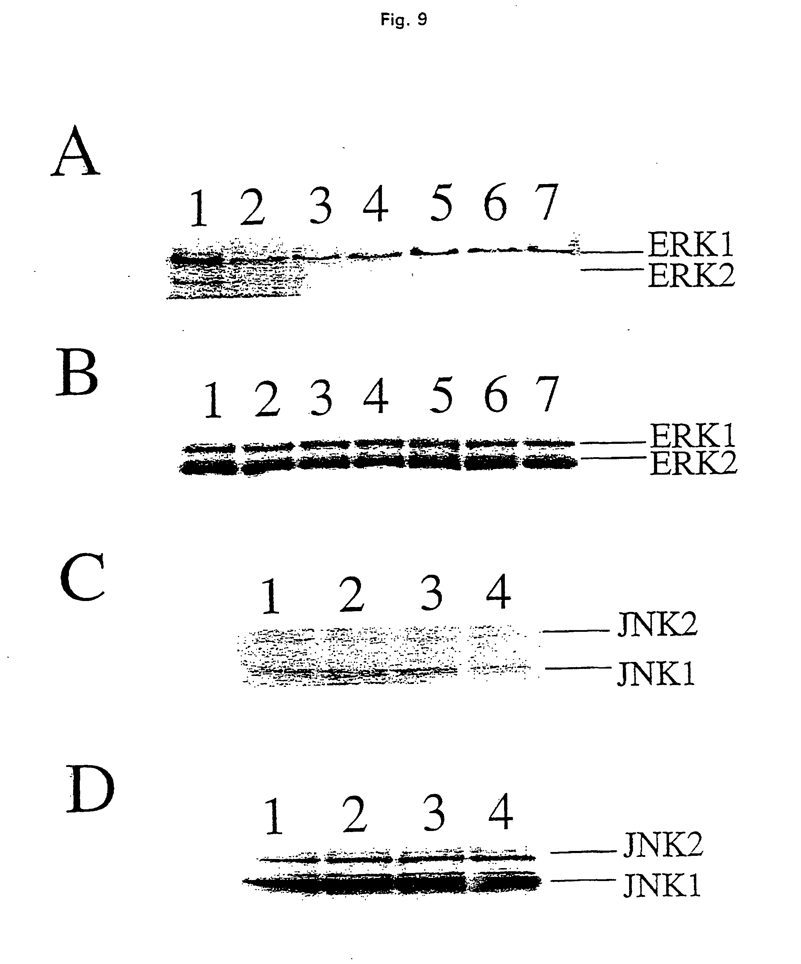

- Fig. 9: shows western blots demonstrating the effect of GDNF and other neurotrophic factors on the phosphorylation of MAP kinase in cultured human corneal stromal keratocytes. Western blots with antibodies against phosphorylated ERK1 (44 kD) and ERK2 (42 kD) (A), total ERK1 and ERK2 (phosphorylated and nonphosphorylated) (44 kD and 42 kD) (B), phosphorylated JNK1 (46 kD) and JNK2 (54 kD) (C), and total JNK1 and JNK2 (phosphorylated and nonphosphorylated) (46 kD and 54 kD) (D) demonstrate that phosphorylation of ERK1 and to a lesser extent of ERK2 was weakly induced by GDNF (lane 5) and NGF (lane 1) in comparison to serum-free control medium (lane 7) or BDNF (lane 3). Phosphorylation of ERK1 by NGF and GDNF was inhibited by addition of the MEK-inhibitor PD 98059 (lane 2 and 6, respectively). Phosphorylation of ERK1 by BDNF remained unchanged upon addition of PD 98059 (lane 4). Phosphorylation of JNK1 (C) was weakly induced by NGF (lane 1), BDNF (lane 2) and GDNF (lane 3) in comparison to serum-free medium (lane 4).

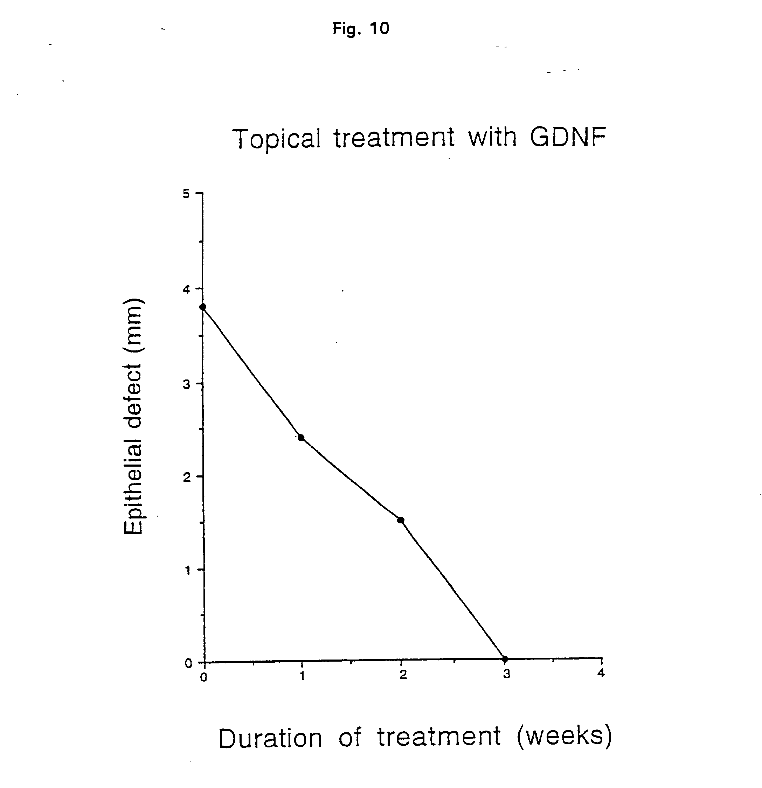

- Fig. 10: is a diagram showing the time course of the size of the epithelial defect in a patient during topical treatment with GDNF.

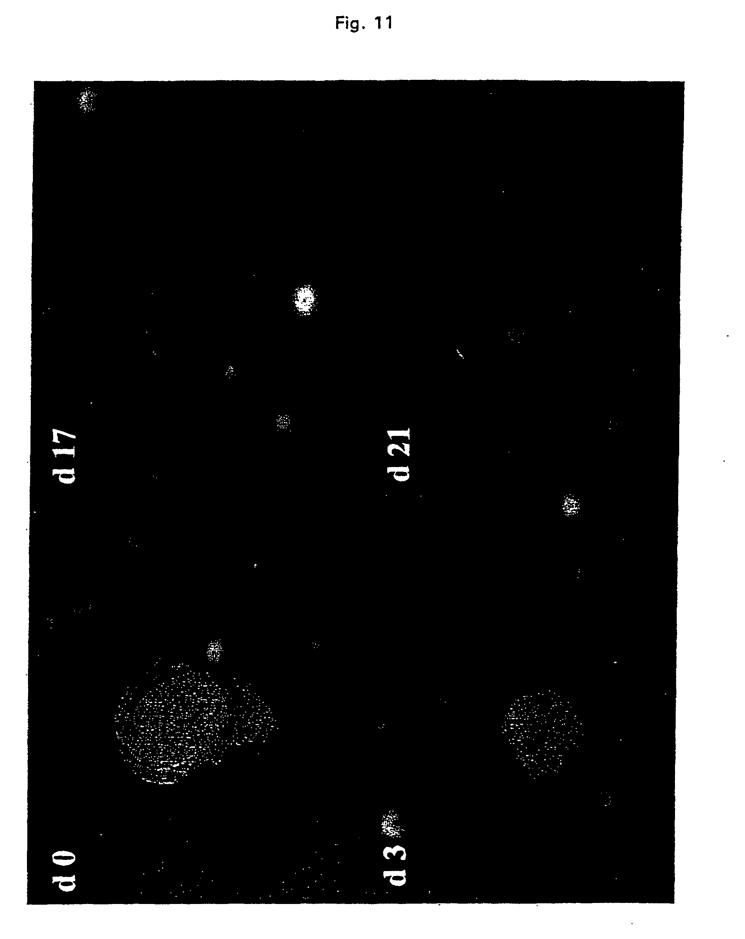

- Fig. 11: shows photographs of the centre of the cornea of the same patient treated with GDNF as in Fig. 10. On the day before starting the treatment with GDNF a large area is visible which can be labelled with yellow fluorescein. This area is not covered by the epithelium (day d0). After three days a significant decrease of the injured area due to the proliferation of the epithelium from above can be observed (d3). On the 17th day only a small central defect which could only be labelled weakly with fluorescein is visible (d17). As soon as 21 days after the beginning of the treatment wound healing is almost completed except for only slight unregularities (d21).

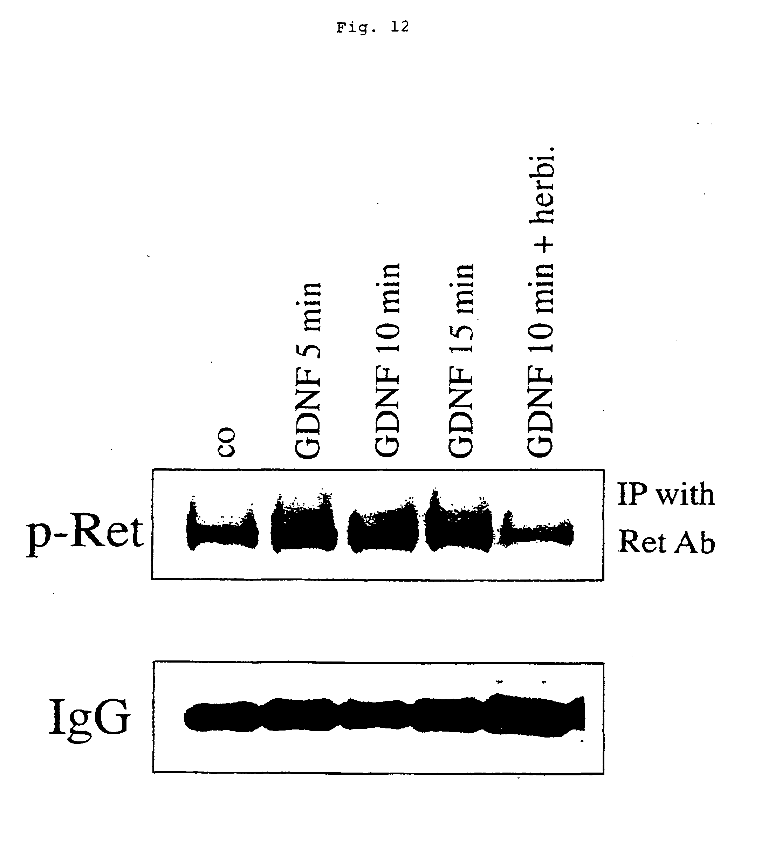

- Fig. 12: is a photograph of western-blot experiments demonstrating the time-dependent tyrosine phosphorylation of Ret by GDNF. The level of phosphorylated Ret (approximately 150kD) in cultured corneal epithelial cells (control) was low prior to addition of GDNF (200ng/ml)(co), increased at 5 minutes and remained on a high level at 10 minutes and 15 minutes after addition of GDNF. Tyrosine phosphorylation of Ret in cells pretreated with herbimycin A and stimulated with GDNF (10 minutes) remains at a lower level than in cells without incubation of herbimycin A. Ig G (immunoglobulin) blot indicates that equal amounts of Ret antibody were used for immunoprecipitation.

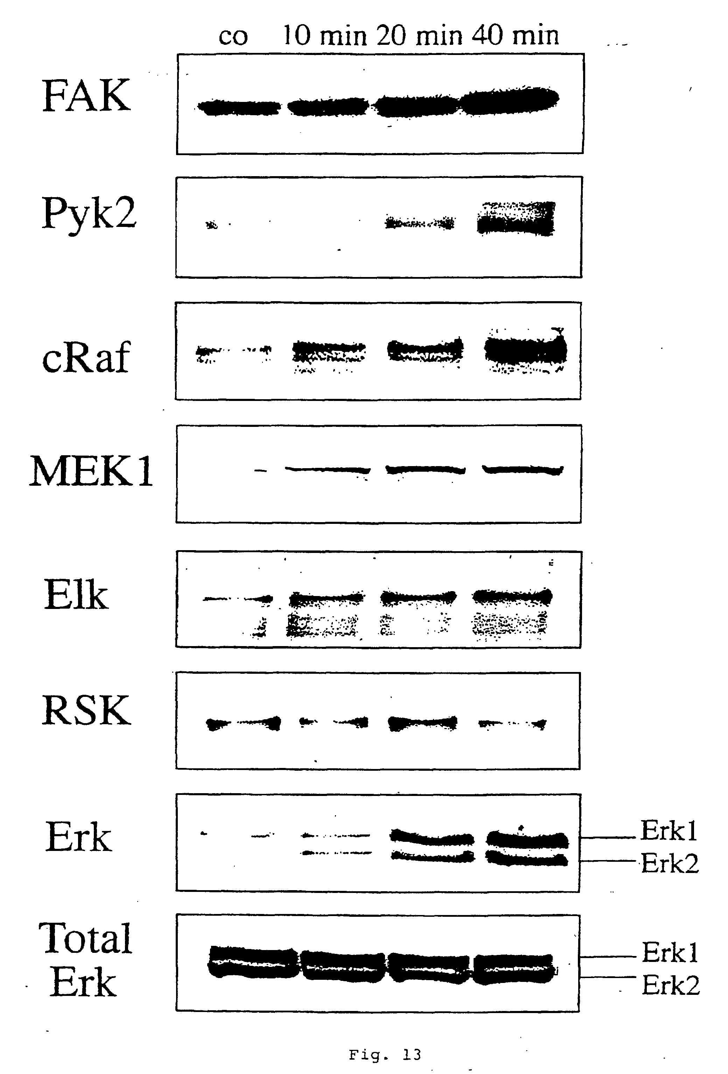

- Fig. 13: is a photograph of western-blot experiments demonstrating the time-dependent phosphorylation of intracellular signals by GDNF. Tyrosine phosphorylation of FAK (approximately 130kD) and Pyk2 (approximately 130kD), serine phosphorylation of cRaf (approximately 80kD), MEK1 (45kD) and Elk (approximately 60kD) as well as tyrosine/threonine phosphorylation of Erk1 (44kD) and 2 (42kD) was induced within 10 minutes after exposure to GDNF and gradually increased over the next 30 minutes. Phosphorylation of p90RSK (90kD) was not induced in response to GDNF stimulation. To show that equal amounts of total protein were loaded in each lane (80 µg) a blot with an antibody against total Erk (phosphorylated and unphosphorylated Erk 1 and Erk 2) is presented.

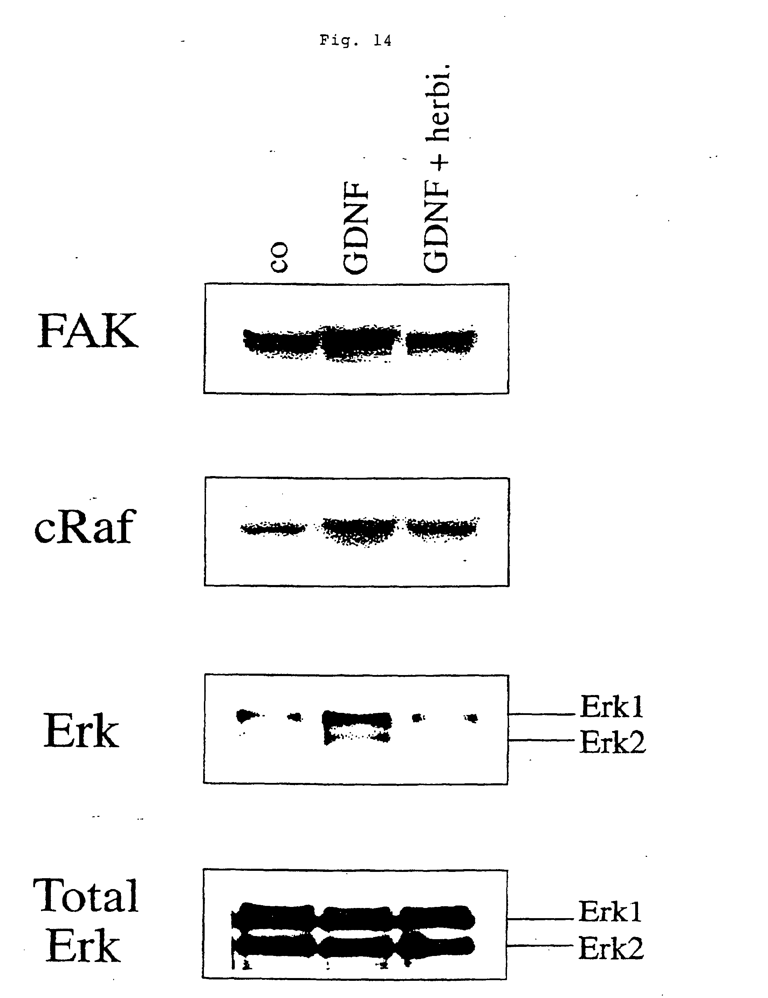

- Fig. 14: is a photograph of further western-blot experiments showing the Inhibition of GDNF-dependent phosphorylation of FAK, cRaf and Erk by herbimycin A. The level of GDNF-dependent tyrosine phosphorylation of FAK, serine phosphorylation of cRaf and tyrosine/threonine phosphorylation of Erk 1 and 2 were all significantly decreased in cultured corneal epithelial cells following preincubation with herbimycin A for two hours. The amount of total Erk (phosphorylated and unphosphorylated Erk 1 and Erk 2) remained unchanged.

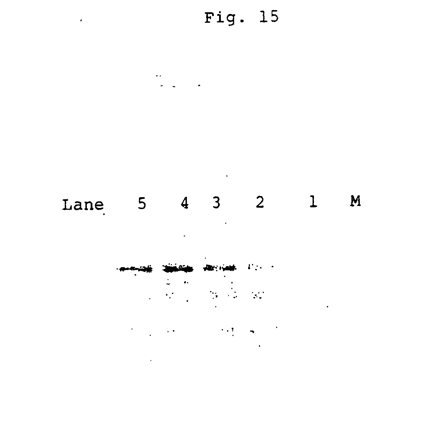

- Fig. 15: is a photograph of a western blot showing the time-dependent phosphorylation of intracellular signals by artemin. Tyrosine phosphorylation of MEK1 (45kD) was very low in control cells (lane 1). Phosphorylation was induced within 10 (lane 2), 20 (lane 3) and more significantly 40 (lane 4) and 60 minutes (lane 5)after exposure to artemin (250 ng/ml). M: molecular weight marker.

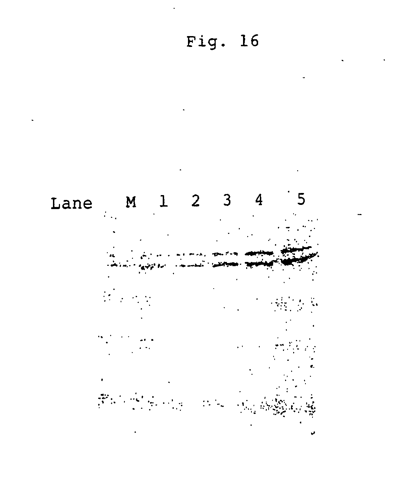

- Fig. 16: is a photograph of a further western blot demonstrating the time-dependent phosphorylation of intracellular signals by artemin. Tyrosine/threonine phosphorylation of Erk 1 and 2 was very low in control cells (lane 1). Phosphorylation was induced within 10, 20 and more significantly 40 and 60 minutes (lanes 2 to 5) after exposure to artemin (250 ng/ml).

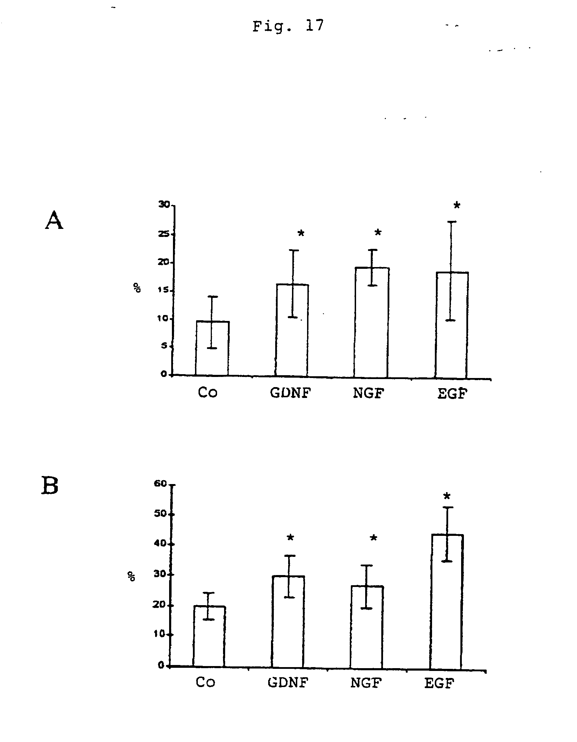

- Fig. 17: shows graphical representations of experiments demonstrating the effect of GDNF on *in vitro* closure of "wounds" in semi-confluent, monolayers. Closure of scratch "wounds" of 1 mm diameter was significantly (*) (p < 0.01) enhanced by 250 ng/ml GDNF in comparison to control cultures (co) in both primary comeal epithelial cells (A) and SV40-transfected comeal epithelial cells (cf. Arraki-Sasaki et al., 1995) (B). The wound closure is expressed as percent of the initial wound gap in representative cultures at 18 hours. NGF and EGF served as postivie controls.

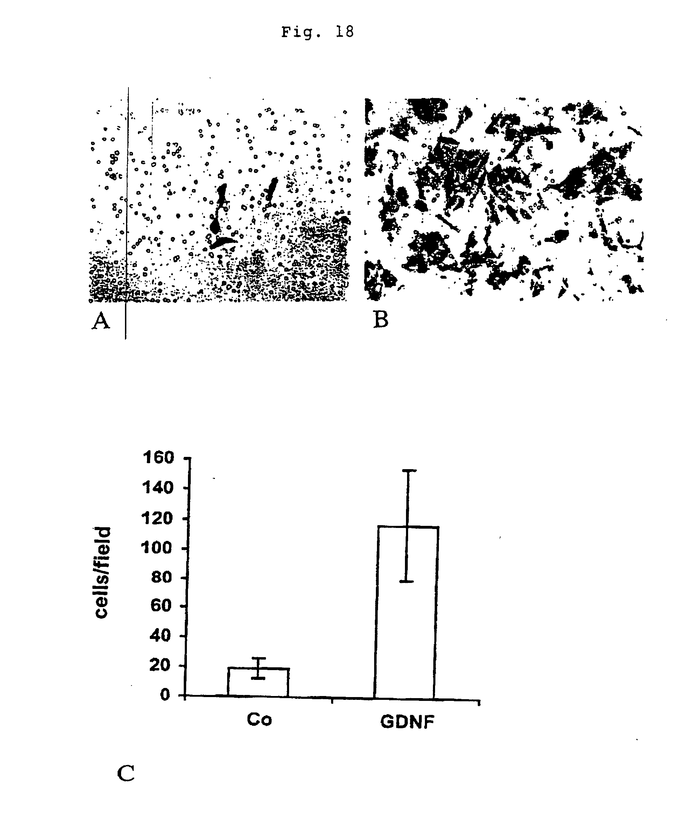

- Fig. 18: shows the results of experiments demonstrating the effect of GDNF on corneal epithelial cell migration in a modified Boyden chamber system. In control medium only few (19 ± 6.8) comeal epithelial cells migrated from the upper chamber through the filter [photohgraph in (A); diagramm in (C)]. Addition of 250 ng/ml GDNF into the lower chamber resulted in a 6 fold increase of cells migration through the filter (117 ± 37.6 ) (p<0.0001) [photohgraph in (B); diagramm in (C)].

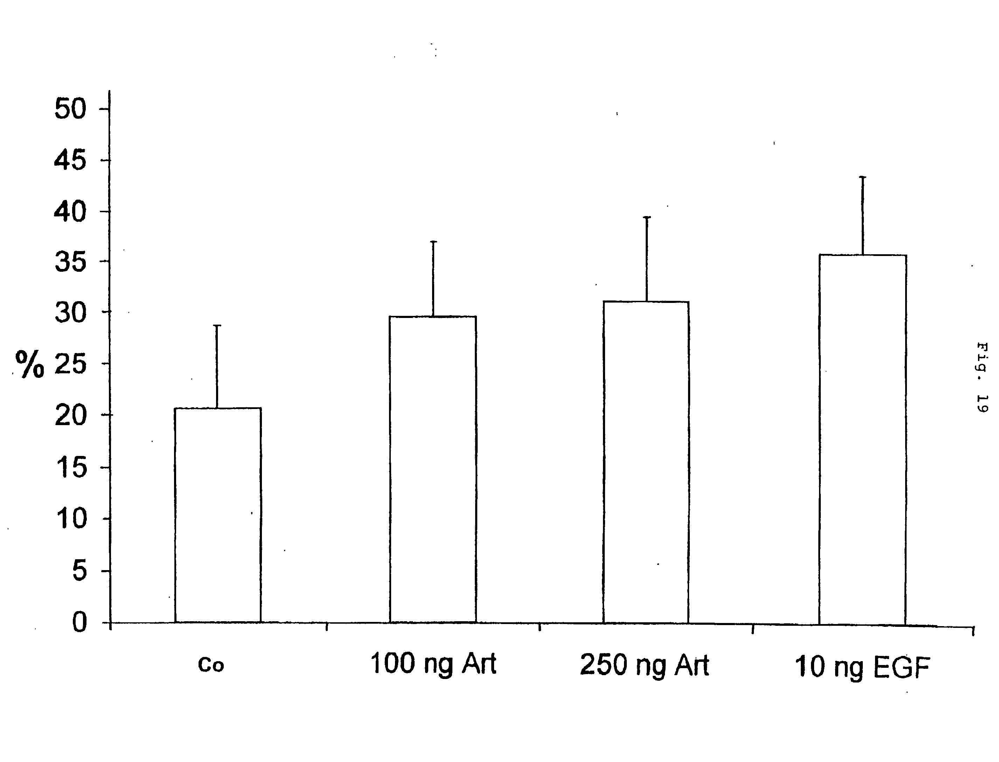

- Fig. 19: is a graphical representation of experiments showing the effect of artemin on in vitro closure of "wounds" in semi-confluent monolayers. Closure of scratch "wounds" of 1 mm diameter was significantly (*) (p < 0.01) enhanced by artemin in concentrations of 100ng/ml and 250 ng/ml in comparison to control cultures (Ko). The results are expressed as wound gap in % in comparison to the original wound gap at the beginning of the experiment in rabbit corneal epithelial cells. The effect of EGF served as a positive control.

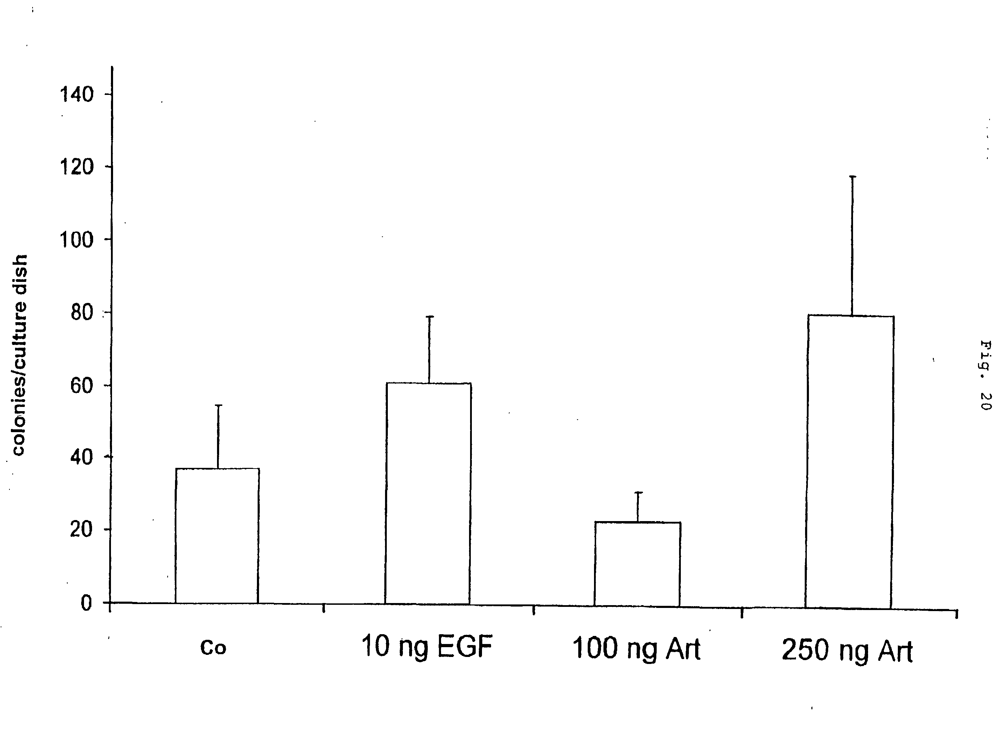

- Fig. 20: is a graphical representation of experiments showing the effect of artemin on *in vitro* proliferation of corneal epithelial cells. Shown are colonies of cells per dish after one week of incubation with either 10 ng/ml EGF as positive control or with artemin in concentrations of 100 or 250 ng/ml in comparison to the control (medium MCDB 151 without growth factors).

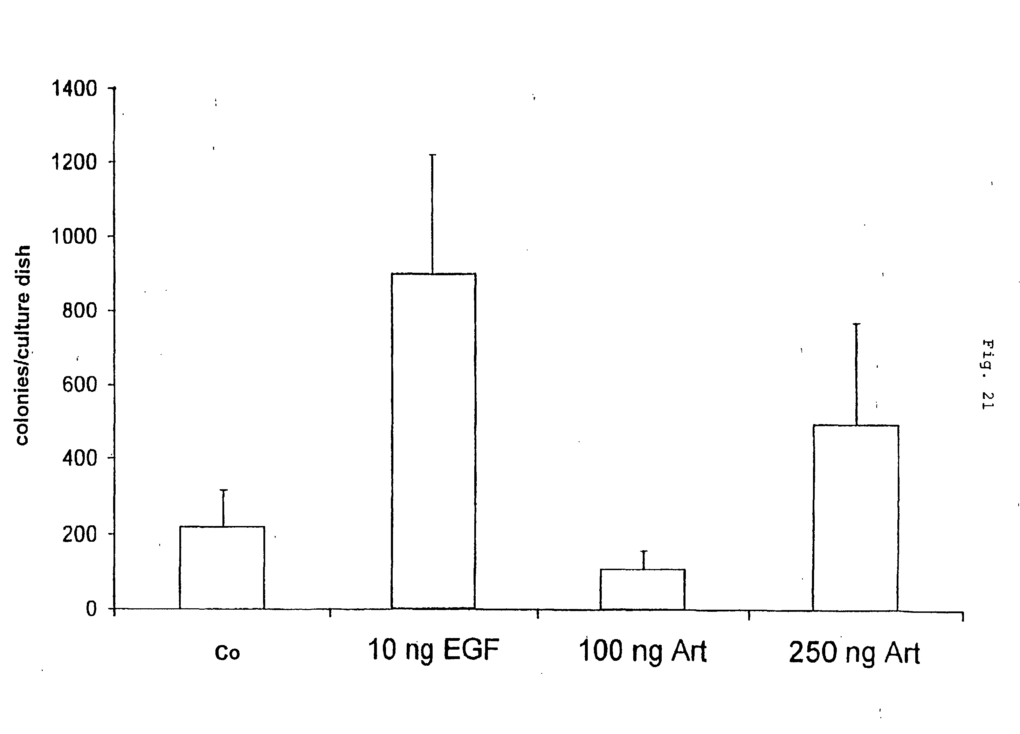

- Fig. 21: is a graphical representation of experiments further illustrating the effect of artemin on *in vitro* proliferation of comeal epithelial cells. Bars represent the number of cells per dish after one week of incubation with either 10 ng/ml EGF as positive control or with artemin in concentrations of 100 or 250 ng/ml in comparison to the control (medium MCDB 151 without growth factors).

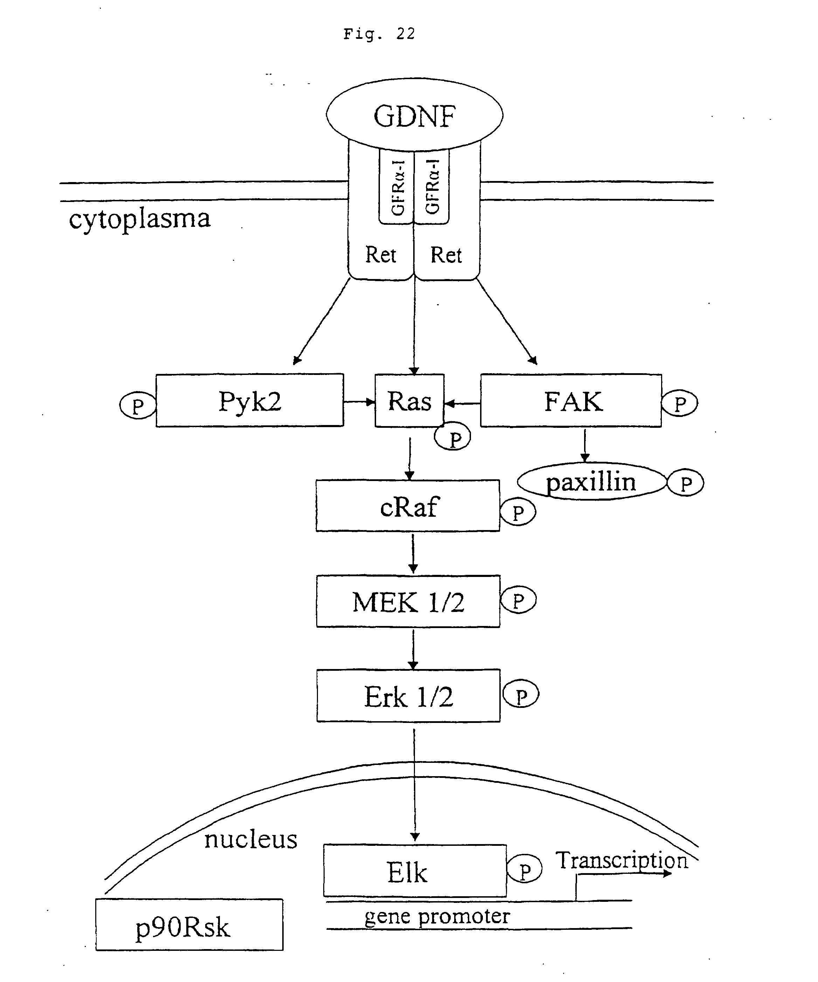

- Fig. 22: is a schematic representation of the signal transduction pathways which appear to be involved in the wound healing processes triggered by GDNF molecules.

The present invention is further illustrated by the following non-limiting example.

### EXAMPLE

### Transcription of GDNF in the human cornea

The transcription of GDNF and other neurotrophic factors was detected in freshly harvested cells from human corneal epithelium (Fig. 2A) and stroma (Fig. 2B) by RT-PCR. In Fig. 2A, the result of a representative RT-PCR shows that the specific cDNA fragment of NGF (lane 1, 233 bp), NT-3 (lane 2, 298 bp), NT-4 (lane 3, 464 bp), BDNF (lane 4, 373 bp) could amplified from *ex vivo* human corneal epithelium. However, transcription of GDNF (lane 5) using the primers listed below could not be detected. This result was confirmed by three independent experiments using cDNA from primary cultured epithelial cells and a human corneal epithelial cell line immortalized with SV40 (Araki-Sasaki et al., 1995). In contrast, *ex vivo* corneal stroma contained mRNA encoding also GDNF (lane 5, 343 bp) (cf. Fig. 2B). However, in this tissue transcription of NT-4 (lane 3) could not be detected using the primers listed below. The results of the RT-PCR experiments were identical when cDNA from cultured corneal stromal keratocytes was used.

All of the above-mentioned PCR products were transformed into *E. coli* and sequenced. Comparison of the resulting DNA-sequences with known genes via the Blast search program of Gen Bank revealed 100% sequence identity with the expected neurotrophic factors in all experiments.

### Transcription of tyrosine kinase receptors specific for GDNF in the human cornea

The *ex vivo* corneal epithelium (Fig. 3A) and stroma (Fig. 3B) also contained mRNA encoding tyrosine kinase receptors which are necessary for binding and signal transduction of neurotrophic factors.

Fig. 3A shows the result of RT-PCR experiments after amplification of cDNA fragments specific for TrkA (lane 1) (570 bp), TrkB (lane 2) (472 bp), TrkC (lane 3) (484 bp) and TrkE (lane 4) (545 bp) from *ex vivo* corneal epithelium. Fig. 3B indicates the same result using mRNA from *ex vivo* comeal stroma. When the cultured corneal epithelial cells (primary cultures or corneal epithelial cell line) or cultured corneal stromal keratocytes were used, the spectrum of RT-PCR was not changed. All the above Trk gene fragments have also been cloned, sequenced and analyzed by the Blast search program for further confirmation.

### Level of transcription of GDNF and corresponding tyrosine kinase receptors in cultured human corneal epithelium and stromal keratocytes

In order to confirm the results of the above PCR experiments as well as to estimate the level of gene transcription, a DNA dot blot analysis was performed (Fig. 4). Since the hybridization probe for the DNA dot blot was first-strand cDNA generated from 1 µg mRNA of cultured epithelial cells or stromal keratocytes, the result of the DNA dot blot allows to estimate and to compare the transcriptional level of GDNF and other neurotrophic factors and corresponding tyrosine kinase receptors in different cells. Fig. 4A shows the spectrum of the transcriptional level of GDNF and other neurotrophic factors and the corresponding tyrosine kinase receptors in the human corneal epithelium cell line. The transcription of NGF (lane 1), BDNF (lane 4) and TrkE (lane 9) was significantly weaker than that of NT-3 (lane 2), NT-4 (lane 3), TrkA (lane 6), TrkB (lane 7) and TrkC (lane 8). The level of transcription of NT-3 (lane 2), NT-4 (lane 3), TrkA (lane 6), TrkB (lane 7) and TrkC (lane 8) was lower than that of GAPDH which was used as positive control (lane 10). GDNF was not transcribed in epithelial cells (lane 5, Fig. 4A) but showed a positive signal in cultured stromal keratocytes (lane 5, Fig. 4B).

### Functional role of GDNF in cultured corneal epithelium and stroma

GDNF had a significant effect on the proliferation of corneal epithelial cells. As shown in Fig. 5 the numbers of colonies per dish increased significantly upon addition of recombinant human GDNF (50 ng, p < 0.05, and 200 ng, p < 0.0001). This indicates that the ability of corneal epithelial cells to form colonies was enhanced by GDNF. Even more important is the effect on the clonal proliferation which is reflected by the number of cells within each colony (Fig. 6). Corneal epithelial cells are continuously entering cellular proliferation which on D6 results in a spectrum of colonies ranging from very small colonies to very large ones. This observation explains the relatively large standard deviation (SD) and the requirement to count a large number of colonies (75) in each dish in order to obtain statistically meaningful data. The clonal proliferation of corneal epithelial cells was significantly stimulated by GDNF (50 and 200 ng/ml, p < 0.01) as shown in Fig. 6.

In addition to the stimulatory effect on comeal epithelial proliferation, GDNF in concentrations of either 20 or 100 ng/ml significantly enhanced the proliferation of stromal keratocytes (p < 0.005) as shown in Fig. 7. These data indicate that GDNF can enhance proliferation of human stromal keratocytes in serum-free medium.

### Effect of GDNF on the phosphorylation of MAP kinase in cultured corneal epithelium and stromal keratocytes

The activation of the MAP kinase signalling cascade is essential for mediating the effect of various growth factors on cellular proliferation and differentiation. Therefore, the intracellular accumulation of phosphorylated MAP kinases ERK and JNK is an indication for the activation of the MAP kinase pathway in response to neurotrophic factors. In order to correlate the accumulation of the signal transduction with the results of the surprising effect of GDNF on the proliferation of cultured corneal epithelial cells and cultured stromal keratocytes, the induction of members of the MAP kinase cascade in human corneal epithelium and stroma was investigated. To ensure that the signals are corresponding to phosphorylation, also the inhibitor PD 98059 was used which inhibits MAP kinases. Fig. 8A shows that-the phosphorylated forms of ERK1 and 2 can be induced in cultured human epithelial cells. As compared to serum-free control medium (lane 7) GDNF at a concentration of 200 ng/ml induced the phosphorylation of ERK1 and ERK2 (lane 5).. This induction was prevented by the addition of the inhibitor PD 98059. The level of phosphorylated ERK1 and ERK2 was also increased by NGF (200 ng/ml) (lane 1), and this effect could also be prevented by PD 98059. Similarily, the level of phosphorylated ERK1 and ERK2 was also increased by BDNF (200 ng/ml) (lane 3), but this increase was not inhibited by PD 98059. The data in Fig. 8B, C and D show the same expression level of total (phosphorylated and non-phosphorylated) ERK1 and ERK2 (Fig. 8B), activated JNK1 and JNK2 (Fig. 8C) as well as total (phosphorylated and non-phosphorylated) JNK1 and JNK2 (Fig. 8D) in human comeal epithelial cells when incubated with or without the above neurotrophic factors. The results indicate that phosphorylation of ERK1 and ERK2 (but not JNK1/2) can be induced by GDNF in cultured rabbit corneal epithelial cells.

Fig. 9 shows that the effect of GDNF on phosphorylation of MAP kinases is different from that of the neurotrophic factors in cultured human corneal stromal keratocytes. The data shown in Fig. 9A indicate that in comparison to the control in stromal keratocytes (lane 7) phosphorylation of ERK1 and to a lesser extent of ERK2 was induced by 200 ng/ml NGF (lane 1), and this increase was inhibited by PD 98059 (lane 2). In contrast, 200 ng/ml BDNF did not induce phosphorylation of ERK1 or ERK2 as compared to the control (lane 3) and remained unchanged with PD 98059 (lane 4). GDNF (200 ng/ml) induced ERK1 to a limited extent (lane 5), and this increase was inhibited by PD 98059 (lane 6). Fig. 9B shows that total ERK was induced to the same level in stromal keratocytes cultured either in serum-free medium or with the above neurotrophic factors. In comparison to ERK1 the expression of both activated JNK1 and 2 in stromal keratocytes was relatively weak. Slight differences could be observed concerning the expression of activated JNK1 which was lower in cells cultured in serum-free DMEM (lane 4) than in cells stimulated with NGF (lane 1), BDNF (lane 2) or GDNF (lane 3). BDNF seemed to have the strongest effect on the activation of JNK1 (lane 2). Activated JNK2 in stromal keratocytes was not induced by neither GDNF, NGF nor BDNF (cf. Fig. 9C). Fig. 9D shows that the expression of total JNK1 and JNK2 is the same in stromal keratocytes regardless of the culture condition. The above results indicate that GDNF, NGF and BDNF have different effects on the accumulation of phosphorylated forms of ERK1 and JNK1 in cultured human stromal keratocytes.

### Summary of in vitro and cell culture data

In order to investigate the effect of GDNF and other neurotrophic factors on human and rabbit corneal epithelium and stroma and the possible transductional pathway involved, transcription of GDNF, NGF, NT-3, NT-4, BDNF, and receptors TrkA to E was investigated by RT-PCR. DNA dot blot analysis allowed an estimation of transcriptional levels. Single cell proliferation assays were performed using recombinant GDNF. MAP kinase signal transduction was investigated by western blot analysis using antibodies against activated and total ERK1/2 and JNK1/2, respectively.

The above results indicate that transcription of NGF, NT-3, BDNF and TrkA, TrkB, TrkC, TrkE receptors can be detected in both *ex vivo* and cultured epithelium and stroma. In contrast, transcription of GDNF was predominantly detected in stroma while transcription of NT-4 was only detected in epithelium. Levels of transcription were higher for NT-3, NT-4 and the Trk receptors and lower for GDNF, NGF and BDNF. GDNF stimulated both epithelial colony formation and proliferation. Stromal proliferation was enhanced by GDNF in serum-free medium. In epithelium predominantly ERK1 was activated by GDNF, NGF and BDNF. In stromal cells GDNF and NGF stimulated phosphorylation of ERK1 and JNK1.

These results show that GDNF is transcribed in the human cornea. GDNF stimulates corneal epithelial proliferation. GDNF has very specific effects on phosphorylation of ERK1 and JNK1 in epithelial and stromal cells. The differential expression of GDNF suggests a regulatory function within the cytokin network of the cornea. The finding that GDNF is predominantly expressed in stromal keratocytes but that it stimulates proliferation of cornea epithelial cells and that the proliferation of stromal keratocytes was effected by GDNF to a lesser extent suggests that GDNF plays its role as an epithelial modulator.

### Therapy of corneal ulcer in a human patient

In order to demonstrate the positive effect on the proliferation of comeal epithelial cells directly, a 45 year old patient suffering from a corneal ulcer with accompanying massive inflammation on the left was treated with recombinant human GDNF. Before the treatment the patient was treated unsuccessfully for six weeks in a hospital specialized on eye diseases with different ointments, eye drops, a contact lens and also with serum drops. Therefore, it was concluded that the patient had to undergo surgery. However, the patient was treated with recombinant human GDNF at a concentration of 0.2 mg/ml (25 µl/dose) in 2 hours intervals for 40 hours, then with 20 µl/dose 6 times a day. As shown in Fig. 10 and 11, respectively, wound healing commenced from day 2 on. From then on wound healing of the epithelium continued and was complete after 3 weeks of therapy (Fig. 10). Moreover, a continuous increase in thickness of the stroma could be observed. Furthermore, after healing of the epithelium a significant reduction of the inflammation as well as of the subjective afflictions was obtained.

### Phosphorylation of Ret is induced by GDNF in a time-dependent manner in cultured corneal epithelial cells

Following stimulation with GDNF, GFRalpha-1 is recruiting Ret to the cell membrane and activates its receptor tyrosine kinase domain to mediate GDNF signals between membrane receptor proteins and intracellular signaling proteins. In order to determine whether Ret is expressed in the comeal epithelium and participating in GDNF-induced signaling pathways, immuno-precipitation using an anti-Ret antibody followed by western blots with monoclonal antibody against phospho-tyrosine was performed in order to detect phosphorylated Ret. Fig. 12 shows that phosphorylated Ret was time-dependently accumulated in comeal epithelial cells in response to GDNF. In comparison to serum-free control cultures (co), tyrosine phosphorylation of Ret was induced within 5 minutes after addition of GDNF (200 ng/ml) to the culture medium. Tyrosine phosphorylation of Ret remained on a high level over 15 minutes. In comparison, the level of phosphorylated Ret in herbimycin-pretreated cells was even lower than in control cultures. These results indicate that GDNF-dependent tyrosine phosphorylation of Ret is specifically inhibited by the tyrosine kinase inhibitor herbimycin A.

### Time-dependent activation of intracellular signals induced by GDNF in cultured corneal epithelial cells

In order to determine which signal transduction pathways are induced by GDNF, it was sought to detect GDNF-dependent tyrosine phosphorylation of FAK family members and GDNF-dependent activation of MAPK signaling components by western blot with specific antibodies against each of the phosphorylated proteins.

Tyrosine phosphorylation of FAK was gradually increased within 40 minutes in the presence of GDNF (Fig 13). Phospho-tyrosine kinase 2 (Pyk2), another member of FAK family, was also phosphorylated within 40 minutes following exposure to GDNF (Fig 13).

Regarding MAPK signaling, cRaf belongs to the MAPK kinase (MKKK) family and plays the role of an initiator for the propagation of MAPK signals. MAPK kinase 1 (MEK1) is a key protein which mediates signaling cascades from MKKK to the two components of MAPK Erk 1 and 2. Activation of Erk facilitates its translocation into the nucleus where it phosphorylates transcription activators such as Elk. Fig 13 demonstrates that all of these signaling components are activated by GDNF: Serine phosphorylation of cRaf, MEK1 and Elk as well as tyrosine/threonine phosphorylation of Erk 1 and 2 were all significantly induced already within 10 minutes after exposure to GDNF and the level of phosphorylation gradually increased over the observation period (Fig 13). In contrast, phosphorylation of RSK in response to GDNF was not detectable (Fig 13). These results indicate that the induction of gene transcription by GDNF is depending on the FAK (Pyk2)-MAPK-Elk pathway.

### Effect of herbimycin A on GDNF-dependent phosphorylation of intracellular signaling proteins

Treatment of cells with the protein-tyrosine kinase inhibitor herbimycin A resulted in a reduction of GDNF-dependent activation of FAK, cRaf and Erk. Western blot analyses show that GDNF-induced phosphorylation of FAK, cRaf and Erk within 30 minutes following addition to the medium (Fig. 14). However, when comeal epithelial cells were cultured in the presence of herbimycin A, GDNF-dependent phosphorylation of FAK, cRaf and Erk was significantly decreased (Fig 14). In this context, it should be to noted that not only tyrosine phosphorylation of FAK but also *serine* phosphorylation of cRaf as well as tyrosine/threonine phosphorylation of Erk 1 and 2 were inhibited by the tyrosine kinase inhibitor herbimycin A. This suggests that activation of cRaf-Erk pathway is largely dependent on phosphorylation of FAK which represents an upstream regulator.

### Phosphorylation of MEK is induced by artemin

Incubation of human corneal epithelial cells with 250 mg/ml artemin induced a time-dependent induction of the phosphorylation of MEK. The western blot in Fig. 15 shows very low level of phosphorylation in the control (lane 1. Incubation with 250 ng/ml artemin for 10, 20, 40 and 60 minutes (laned 2 to 5) led to an increasing amount of phosphorylated MEK in corneal epithelial cells. All lanes in Fig. 15 were loaded with the same amount of protein.

### Phosphorylation of Erk 1/2 is induced by artemin

Incubation of human corneal epithelial cells with 250 mg/ml Artemin induced a time-dependent induction of the phosphorylation of Erk 1/2. The western blot in Fig. 16 shows a very low level of phosphorylation in the control (lane 1). Incubation with 250 ng/ml artemin for 10, 20, 40 and 60 minutes (lanes 2 to 5) gradually increased the amount of phosphorylated Erk 1 and 2 in comeal epithelial cells. All lanes in Fig. 16 were loaded with the same amount of protein.

### GDNF induces migration of corneal epithelial cells

Following a scratch "wound" of about 1 mm diameter in a subconfluent monolayer of primary human corneal epithelial cells in control medium [SHEM without additives (Co)], less than 10 % of the gap in the cell monolayer was filled with cells within 18 h (Fig 17A shows the result of a representative culture experiment). The addition of both GDNF or NGF as well as EGF resulted in a significant increase of in vitro wound healing. As shown in Fig. 17A, 16.5 ± 6 % of the gap was filled in the presence of 250 ng/ml GDNF which represents a significant increase over the control (p < 0.01).

Similarly, exposure of cells to NGF (250 ng/ml) or to EGF (10 ng/ml) resulted in about 20% closure of the gap (19.6 % ± 3.1 and 19.0 % ± 8.6 respectively). These data indicate that NGF and EGF also significantly promote closure of the wound in comparison to the control (p< 0.0001 and p< 0.0001, respectively).

These results were confirmed by using corneal epithelial cells from the SV-40 transformed cell line as shown in Fig. 17B. In control medium [SHEM without additives (Co)] 20% ± 4.5 of the gap in the cell monolayer was filled after 18 h. In the presence of GDNF (250 ng/ml) 30% ± 6.9 of the gap was filled which indicates a significant increase as compared to the control (p < 0.001). Similarly, exposure of cultures to either NGF (250 ng/ml) or EGF 10 ng/ml) showed that 27 % ± 7.1 and 44.6 ± 9 % of the gap in the cell layer had been filled with cells (p<0.0001 and p<0.0001, respectively) (Fig. 17B). These results demonstrate that GDNF significantly enhanced closure of a "wound" in a semiconfluent monolayer of corneal epithelial cells and that this effect was similar to that of NGF and EGF.

In order to further confirm the effect of GDNF on cell migration, a modified Boyden chamber analysis was performed: In control medium (SHEM without growth factors) only few cells (19 ± 6.8) migrated through a filter of 8 µm pore size (Fig. 18A,C). However, when 250 ng/ml GDNF was added to the lower well of the modified Boyden chamber, the number of cells which had migrated through the pores of the filter increased more than 6 fold (117 ± 37.6) (p< 0.0001); cf. Fig. 18B,C.

### GDNF-induced migration is inhibited by Herbimycin A

In order to further test the significance of FAK-MAPK-signaling pathways during in wound healing, the effect of Herbimycin A on GDNF-mediated cell migration was studied in the *in vitro* wound healing model. Herbimycin A was capable to block cell migration in the presence of GDNF. In a representative culture containing 250 ng/ml GDNF 37.9 ± 8.1 % of the wound gap was filled with cells after 18 hours. In the presence of GDNF and 10 µM Herbimycin A only 19.2 ± 5.5 % of the gap was closed (p < 0.001).

### Artemin induces migration of corneal epithelial cells

Following a scratch "wound" of about 1 mm diameter in a subconfluent monolayer of primary rabbit corneal epithelial cells in control medium [medium 500 without additives) (Co)] about 20% of the gap in the cell monolayer was filled with cells within 6 h (Fig. 19 shows the result of a representative culture experiment). The addition of both artemin (100 or 250 ng/ml) or EGF (10 ng/ml) resulted in a significant increase of in vitro wound healing. As shown in Fig. 19, 29 ± 8 % of the gap was filled in the presence of 100 ng/ml artemin which represents a significant increase over the control (p < 0.01). Similarly, exposure of cells to 250 ng/ml artemin resulted in about 30% to 35% closure of the gap. These data indicate that artemin also significantly promotes closure of the wound in comparison to the control (p< 0.001).

### Artemin enhances the formation of colonies and thereby stimulates proliferation

Fig. 20 shows the effect of artemin (100 ng/ml and 250 ng/ml) on the total number of colonies of rabbit corneal epithelial cells on day 6. Control cultures (Co) contained a mean of 40±20 colonies. Cultures incubated with 10 ng/ml contained a mean of 58±18 colonies (p < 0.01). Addition of 250 ng/ml artemin significantly increased the number of colonies over the control to a mean of 79±38 cultures (p < 0.01).

### Artemin increases the total number of cell per dish and thereby induces proliferation of corneal epithelial cells

Fig. 21 shows the number of cells in response to artemin. In control cultures, a total of 200±98 cells was present. In contrast, the addition of 250 ng/ml artemin significantly increased the total number of cells per dish (p < 0.001). These data clearly demonstrate that Artemin enhances the proliferation of corneal epithelial cells *in vitro.*

### Methods

### Ex vivo cornea tissue

Fresh *ex vivo* corneal epithelium and stroma was obtained from 8 eyes undergoing enucleation for choroidal melanomas following informed consent and in conformity with the tenets of the Declaration of Helsinki. Immediately following enucleation all layers of the central and mid-peripherial corneal epithelium within an area of approximately 8 mm were removed by mechanical scraping. Within this area small samples of the stroma were excised with a diamond blade. Tissue samples were shock-frozen.

### Cell culture

Human cornea stored for less than 24 h in Likorol® (Chauvin-Opsia, Ablege Cedex, France) at 4°C were obtained through the eye bank of the Department of Ophthalmology, University of Heidelberg, Medical School, Heidelberg, Germany. All corneas were of transplant quality but excluded from clinical use because of non-ocular reasons according to international eye bank criteria. Both epithelial and stromal cells were cultured on plastic dishes as outgrowth cultures with slight modification of the technique described in You et al. (1999). For RNA extraction explant cultures were initiated and cultured in SHEM medium [1:1 mixture of Dulbeco's modified Eagle's medium and Ham's nutrient mixture F10 with 10% fetal bovine serum (FBS), Gibco, Grant Island, NY, USA], 5 µl insulin and 10 ng/ml EGF without antibiotics (cf. Arrak-Saki et al., 1995; Shimura et al., 1997). The epithelial phenotype of cultures was confirmed by staining with an antibody specific for cytokeratin K12. Since this method only yielded a very limited amount of cells we also used a SV 40 adenovirus-transformed corneal epithelial cell line (described in Arrak-Saki et al., 1995). Similar to normal comeal epithelium these cells exhibit clonal growth characteristics and display a comeal epithelial phenotype including, for example, expression of keratin K12. The cell line was cultured in SHEM medium as described in Arrak-Saki et al. (1995) and Shimura et al. (1997). Stromal fibroblasts were cultured in DMEM + 10% FBS as described in You et al. (1999). All experiments were performed in triplicate and with cells obtained from different donors.

### Isolation of total RNA and mRNA purification

Total RNA was isolated according to the guanidinium thiocyanate phenol-chloroform extraction method (Chomczynski et al., 1987) by use of a Promega RNAgents® total RNA isolation system kit (Promega Co. Madison WI, USA) as described in You et al. (1990). For mRNA isolation a Promega polyATract® system III was used as described in You et al. (1990). In order to minimize the risk of contamination by genomic DNA mRNA samples were digested by RNase-free DNase followed by phenol-chloroform-isoamyl alcohol extraction and isopropanol precipitation.

### PCR primers and reverse transcription-polymerase chain reaction (RT-PCR)

For PCR primer design known coding sequences were taken from GenBank. Due to the high structure similarity of the sequences of all known members of the neurotrophin gene families and the neurotrophin tyrosine kinase receptor family, all sequences in open reading frames were compared using a multiple sequence alignment program as described in You et al. (1999). Whereever possible primers were designed to span one or more introns of the genomic sequence: NGF: sense GAGGTGCATAGCGTAATGTCCA (SEQ ID NO 6), and antisense TCCA-CAGTAATGTTGCGGGTCT (SEQ ID NO 7) (product of 233 bp) (GenBank accession no.: V 0511, X 52599); NT-3: sense TTACAGGTGACCAAGGTGATG (SEQ ID NO 8), and antisense GCAGCAGTGCGGTGTCCATTG (SEQ ID NO 9) (product of 298 bp) (GenBank accession no.: M 37763); NT-4: sense, CTCTTTCTGTCTCCAGGTGCTCCG (SEQ ID NO 10), and antisense CGTTAT-CAGCCTTGCAGCGGGTTTC (SEQ ID NO 11) (product of 464 bp) (GenBank accession no.: M 86528); BDNF: sense GTGAGTTTGTGTGGACCCCGAG (SEQ ID NO 12) and antisense CAGCAGAAAGAGAAGAGGAGGC (SEQ ID NO 13) (product of 373 bp) (Genbank accession no.: X 60201, X 91251); GDNF: sense GCCCTTCGCGTTGAGCAGTGAC (SEQ ID NO 14) and antisense CTCGTACGTTGTCTCAGCTGC (SEQ ID NO 15) (product of 343 bp) (GenBank accession no.: NM 000514); TrkA: sense GATGCTGCGAGGCGGACGGC (SEQ ID NO 16) and antisense CTGGCATTGGGCATGTGGGC (SEQ ID NO 17) (product of 570 bp) (GenBank accession no.: M 23102); TrkB: sense TGCACCAAC-TATCACATTTCTCG (SEQ ID NO 18) and antisense CACAGACGCAATCAC-TACCCA (SEQ ID NO 19) (product of 472 bp) (GenBank accession no.: S 76473); TrkC: sense ACTTCGGAGCATTCAGCCCAGAG (SEQ ID NO 20) and antisense ACTCGTCACATTCACCAGCGTCAA (SEQ ID NO 21) (product of 484 bp) (GenBank accession no.: S 76475, U 05012); TrkE: sense AGGAGTACTT-CAGGTGGATC (SEQ ID NO 22) and antisense ACTGGAGAAGCTGTGGTTGCT (SEQ ID NO 23) (product of 545 bp) (GenBank accession no.: X 74979).

The first-strand cDNA was synthesized as described in You et al. (1999). PCR was performed using 0.5 µl of single-strand cDNA with 3 units of *Thermus aquaticus* (Taq) DNA Polymerase, mixture of deoxyribonucleotides (in a final concentration of 0.2 mM), 10 x PCR buffer (5 µl) and 25 pmol of sense and antisense primers in a total volume of 50 µl (all reagents were from Takara Shuzo Co., Ltd., Japan). The final concentration of MgCl₂ in the buffer was 1.5 mM. A PTC-100 programmable thermocycler (MJ Research, Watertown, MA, USA) was used at 95°C for 3 min (predenaturation). Then 35 cycles were performed including denaturation at 94°C for 1 min, annealing at 55°C for 1 min and extension at 72°C for 1 min.

The PCR products were size-fractionated by agarose gel electrophoresis using 1.8% agarose/1 x TAE gels stained with 0.5 µg/ml ethidium bromide. All PCR fragments were cloned into pCR 2.1 vector (Invitrogen Corp., San Diego, CA, USA) and sequences were confirmed by standard methods.

### DNA dot blot analysis for detection of the level of gene transcription of cultured cornea

In order to estimate the level of transcription in cultured epithelial stromal cells a DNA dot blot analysis was performed. Since it was not possible to culture sufficient quantities of human corneal epithelial cells, a corneal epithelial cell line was used as a source of corneal epithelium. Cloned PCR fragments corresponding to a neurotrophic factor family and Trk receptor genes were amplified using the above mentioned primers and purified from agarose gels. 0.1 µg PCR product was loaded onto nylon membranes as dot. In order to generate the high dosage probe 1 µg mRNA was isolated from cultured epithelial and stromal cells and transcribed with a digoxygenin probe synthesis mix (from Boehringer Mannheim, Mannheim, Germany) for the synthesis of degoxygenin-labeled first-strand cDNA. The DNA blots were then prehybridized and hybridized with the digoxygenin-labelled cDNA probe in DIG EasyHyb Buffer, (Boehringer Mannheim) at 40°C overnight. After post hybridization washing, the blots were treated with the DIG washing kit from Boehringer Mannheim according to the manufacturers description and exposed to ECL-film (Amersham Life Science, Little Chalfont, UK). For comparison, a cDNA fragment encoding reduced glyceraldehyde-phosphate dehydrogenase (GAPDH) was used as positive control.

### Investigation of components of the MAP kinase signal transduction pathways induced by neurotrophic factors in cultured cornea

In order to evaluate the effect of GDNF and other neurotrophic factors on the activation of signal transduction pathways in cultured corneal epithelial and stromal keratocytes, western blots were performed for detecting an accumulation of phosphorylated MAP kinases ERK and JNK in the presence of GDNF, NGF and BDNF. Human stromal keratocytes were cultured in RPMI 1640 medium containing L-glutamine (glutaMAX) or DMEM with 10% FBS for one day and starved in serum-free medium for another day. Cultures were then washed with PBS and incubated in serum-free DMEM without additives or with recombinant human GDNF (200 ng/ml), recombinant human NGF (200 ng/ml) and recombinant human BDNF (200 ng/ml) (all obtained from R & D Systems, Mineapolis, MN, USA) for 30 min. Some cultures were incubated with an inhibitor of MAP kinases (PD 98059, Torcris Cookson Ballwin, MO, USA) at 100 µM for 1 h prior to exposure to neurotrophines. After washing with PBS, culture cells were solubilized in lysis buffer containing 50 mM Tris-CI (pH 8.0), 150 mM NaCI, 0.02% sodium azide, 100 µg/ml PMSF, 1% Triton X-100 and a mixture of several protein inhibitors (Complete®, Boehringer Mannheim) (one tablet/50 ml buffer). 50 µg total protein per lane was fractionated by a 10% STS-MOPS NUPAGE Bis-tris gel (NOVEX, San Diego, CA, USA) and blotted onto nitrocellulose membrane. Membranes were stained with diluted polyclonal antibodies against ERK1, ERK2, JNK1 and JNK2 (Santa Cruz Biotechnology, Santa Cruz, CA, USA). Also a polyclonal antibody was used which recognizes the deactivated form of either ERK1 and ERK2 which was raised against the catalytic core of the phosphorylated threonine residue 183 and tyrosine residue 185 of mammalian ERK2. Similarily, a polyclonal antibody recognizing the phosphorylated form of JNK1 and JNK2 was used (both from Promega). In the last step the membranes were visualized with the ECL western blot analysis system (Amersham).

### Investigation of the effect of GDNF on proliferation of corneal epithelial and stromal cells

In order to evaluate the effect of GDNF on corneal proliferation, recombinant human GDNF was used (R&D, Minneapolis, MN, USA). For the evaluation of the effect on comeal epithelial proliferaton a single cell clonal growth model was utilized which allows to determine the effect of the given growth factor on both colony formation as well as clonal expansion (Kruse et al., 1991). New Zealand white rabbits were housed and treated according to the ARVO Resolution of Animals in Research and under observation of German Federal Laws and the laws of the State of Baden-Württemberg. Prior to sacrifice with an intravenous overdose of pentobarbital, they received an intramuscular injection of xylazine hydrochloride and ketamine hydrochloride. The details of the clonal growth assay are described in Kruse et al. (1991). 5000 viable cells were seeded in each 16 mm dish in serum-free medium MCDB 151 with a supplement (S) of insulin (5 µg/ml), transferrin (5 µg/ml), selenium (5 ng/ml) and hydrocortisone (5 µg/ml) (all from Sigma, Deisenhofen, Germany). This seeding density resulted in a single cell clonal growth which could be quantified under the phase contrast microscope (on day 6) by determination of the number of colonies per dish as well as the number of cells per colony. This quantification was facilitated by the use of dishes which contained a grit on the bottom which is roughly 2 mm wide (from Sarstedt, Newton, NC, USA). For data collection the entire surface area of four randomly selected dishes for each condition was screened. Furthermore, the number of cells per colony was determined in 75 randomly selected colonies for each condition. Furthermore, the total number of cells/dish was calculated in order to carry out an evaluation for artemin. In order to stimulate the cellular proliferation, GDNF (50 or 200 ng/ml) or artemin (250 ng/ml) was added to the medium. In order to estimate the rate of proliferation after 12 days (a time when neighbouring colonies started to grow into each other and, therefore, prevented numerical quantification) dishes were fixed in -20°C methanol and stained with methylene blue.

In order to investigate the effect of GDNF on the proliferation of cultured stromal keratocytes the cells were passaged from DMEM +10% FBS into DMEM +1% FBS or into DMEM without FBS at a density of 5 x 10⁴ cells/60 mm dish. Some cultures received recombinant human GDNF at the above-mentioned concentrations. Proliferation was measured after 6 days by counting cells under the phase contrast microscope (50 fields at 100 x magnification per condition) as well as trypsinized cells. Also a Cell Titer 96 AQueous One Solution proliferation assay (Promega) was performed according to the manufacturess description. For this colorimetric assay 500 or 1000 cells were grown for 6 days in 96-well-plates (Falcon). Upon addition to the culture well a titrazolium dye is bioreduced by cells into a colored formazan product and the absorbance is quantified at 490 nm. The quantity of the formazan product is proportional to the number of living cells in the well and can therefore serve to estimate proliferation.

### Investigation of signal transduction pathways induced by GDNF and artemin in corneal eptithelial cells

In order to investigate which protein kinase cascades are activated by GDNF and artemin, corneal epithelial cells from the cell line were seeded at a density of 5 x 10⁵ cells/75 cm² and cultured in SHEM with 10% FBS for one day. Cultures were then washed with PBS and incubated in serum-free SHEM without additives or with GDNF (200ng/ml) or with artemin (250 ng) for 10 to 40 minutes. After washing with PBS, cultured cells were solubilized in lysis buffer containing 50 mM Tris₂Cl (pH8.0), 150 mM NaCI, 0.02% sodium azide, 100 µg/ml phenylmethylsulfonyl fluoride, 1 mM Na₃VO₄, 1% Triton X-100 and a mixture of several protease inhibitors [completeTM, Boehringer Mannheim (1 tablet/30 ml buffer)]. 80 µg total protein per lane was fractionated by NuPAGE 10% SDS-MOPS-Bis-Tris gel or NuPAGE 3-8% Tris-acetate gel (all from NOVEX, San Diego, CA) and blotted onto a nitrocellulose membrane. Phosphorylated proteins were detected with phospho-specific antibodies and visualized with the ECL western blot analysis system (Amersham). Antibodies against phospho-Raf(ser259), phospho-MEK1/2(ser217/221), phospho-MAPK (Erk1/2) (thr202/tyr204), phospho-p90 ribosomal S6 kinase (RSK) (ser381) and phospho-Elk-1(ser383) were obtained from New England Biolabs (Beverly, MA). Antibody against phospho-FAK (tyr397/tyr407/tyr576/tyr577/tyr861/tyr925) and phospho-Pyk2 (tyr402/tyr579/tyr/580/tyr881) were purchased from Biosource (Camarillo, CA, USA). A monoclonal antibody against phospho-tyrosine came from Sigma (Deisenhofen, Germany).

Immuno-precipitation was carried out for detecting the tyrosine phosphorylation of Ret and paxillin. In brief, 1ml lysate of 5 x 10⁵ cells (cultured under various culture conditions) was incubated with 40 µl protein-G-agarose (Boehringer Mannheim) for 2 hours followed by a brief centrifugation at 12000 rpm. The supernatant was incubated with 40 µl protein-G-agarose and 10 µg polyclonal antibody against Ret (Santa Cruz) or paxillin (Sigma) overnight at 4°C with agitation. Protein G-agarose complex was then collected by brief centrifugation and washed in lysis buffer for two times. The bound protein pellet was eluted in SDS gel-loading buffer by boiling and proteins were separated by NuPage gel electrophoresis and blotting to a nylon membrane followed by visualization as described above.

### In vitro wound heating assay and modified Boyden chamber analysis

In further experiments the effect of GDNF on corneal epithelial cell migration and wound healing was studied in an *in vitro* wound healing model as well as in a modified Boyden chamber assay.

For *in vitro* wound healing assays primary corneal epithelial cells and cells from the corneal epithelial cell line were seeded at a density of 5 x 10⁵ onto 60 mm plastic dishes with 2 mm grid (Sarstedt, Nümbrecht, Germany) and cultured for 2 days until subconfluency. For experiments with artemin primary cultured rabbit comeal epithelial cells were used. These cells had been isolated with Dispase (1,2 U/ml) and trypsin/EDTA. Rabbit cells had been cultured in medium 500 with additives (Cascade Biologies).

The cell layer was injured under the microscope by inducing several parallel scratches with a soft plastic cell scrubber (Falcon, Becton and Dickinson, Heidelberg, Germany). The tip of the scrubber was cut and measured about 1 mm and consequently the width of the scratch in the cell layer was also about 1 mm. Selected areas in the dishes were marked with a very fine marker pen and consecutive images were taken at different time points at 5 x magnification under an inverted phase contrast microscope (Axiovert 25, Zeiss, Jena, Germany) equipped with a video camera. Following the experimental injury dishes were incubated in SHEM medium either containing no growth factors (control) or containing either GDNF (250 ng/ml) (recombinant human GDNF expressed in *E. coli*), NGF (250 ng/ml) (Boehringer Mannheim, Germany) or EGF (10 mg/ml). For experiments investigating artemin cells were cultured in medium 500 without additives and 250 ng artemin was added. For each condition four representative areas were evaluated within three dishes. The mean diameter of the scratch in each representative area was set as 100% at the beginning of the experiment. 18 hours later the mean diameter of the same scratch was calculated again and expressed in percent of the diameter at the beginning of the experiment.

In order to determine which signal transduction pathways are mediating the effect of GDNF on corneal epithelial migration in this model, the tyrosine kinase inhibitor herbimycin A was used as described above.

In order to further evaluate the chemotactic effect of GDNF on corneal epithelial cells, a modified Boyden chamber assay was used. 4 x 10⁵ cells from the either primary cultured cells or the corneal epithelial cell line were seeded onto tissue culture inserts containing a polyethylene terephthalate (PET) filter having a pore size of 8 µm (Falcon). Within 4 hours after seeding most cells had attached to the filter and formed a semiconfluent monolayer. The medium was then changed to SHEM without additives in the upper well and SHEM with GDNF (250 ng/ml) in the lower well. After 24 h of culture cells were removed from the surface of the insert by gentle scrubbing with a rubber policeman. Cells on the bottom of the insert (which had migrated through the filter) were fixed with -20° C methanol and stained with crystal violet. The entire surface of the filters was screened for cells under the microscope.

### Statistical analysis

All experiments examining the effect of GDNF on corneal proliferation were performed in triplicate with cells from different donors. The influence of growth factor on colony formation, colony size and cell number was studied using one-way analysis of variance. The log transformation was used as necessary to affect homogeneity of variance and normality in these data. Student's t-test was employed to determine which differences were significant after analysis of variance.

### Patient

Age 45 years. Comeal ulcer accompanied by massive inflammation on the left. Epithelial defect of 3.8 x 3.0 mm in size, furthermore ulcus of corneal stroma in form of a punched-out defect comprising 80% of the thickness of the cornea. No pain sensation of the cornea and significant accompanying inflammation of the eye.

### Therapy

The patient was treated with recombinant human (rh) GNDF (0.2 mg/ml) (25 µl/dose) in 2 hours intervals for 48 hours, then the doses were reduced to 20 µl 6 times per day. After healing of the epithelium the concentration was reduced to 0.1 mg/ml, and 20 µl doses were given 5 times per day. For adminstration, lyophilized recombinant human GDNF was diluted in sterile balanced salt solution (Alcon, Freiburg Germany).

### References

Arraki-Sasaki et al. (1995) *Invest. Ophthalmol. Vis. Sci.* 36, 614-621.

Chomczynski et al. (1987) *Anal. Biochem*. 162, 156-159.

Kruse et al. (1991) *Invest. Ophthalmol. Vis. Sci.* 32, 2086-2095.

Lambiase et al. (1998) *N. Engel. J. Mit. 33,* 1174-1180.

Shimmura et al. (1997) *Invest. Ophthalmol. Vis. Sci.* 38, 620-626.

You et al. (1999) *Invet. Ophthalmol. Vis.* Sci. 40, 296-311.

## Claims

1. Use of a glial cell line-derived growth factor (GDNF) or a functionally active derivative or part thereof and/or an agonist which substitutes the functional activity of GDNF, and/or a nucleic acid containing at least a nucleotide sequence encoding the primary amino acid sequence of GDNF or the functionally active derivative or part thereof and/or of the agonist for the manufacture of a pharmaceutical composition for epidermal and stromal wound healing and/or for the treatment of epidermal and stromal wound healing disorders and/or scarring disorders.

2. The use of claim 1, wherein the pharmaceutical composition comprises human recombinant GDNF or a functionally active derivative or part thereof.

3. The use of claim 1, wherein the pharmceutical composition comprises a nucleic acid containing at least the nucleotide sequence encoding human wild-type GDNF.

4. The use according to anyone of claims 1 to 3, wherein the pharmaceutical composition contains at least one further agent having a trophic effect on epithelial and/or neuronal cells.

5. The use of claim 4, wherein the agent is selected from the group consisting of TGF-βs, BMPs, GDFs, activin/inhibin, neurotrophins, EGF, HB-EGF, TGF-α, FGFs, KGF, HGF, IGFs, PDGFs, fibronectin and metabolites thereof.

6. The use according to anyone of claims 1 to 5, wherein the pharmaceutical composition contains at least one further agent having an anti-inflammatory effect.

7. The use according to claim 6, wherein the anti-inflammatory agent is selected from the group consisting of analogs of cortisone, inhibitors of the NF-κB pathway, inhibitors of the arachidonic acid pathway and antibodies against chemokines.

8. The use according to anyone of claims 1 to 7, wherein the pharmaceutical composition comprises cells which produce GDNF or the functionally active derivative or part thereof and/or the agonist thereof.

9. The use according to anyone of claims 1 to 8, wherein the wound is in the anterior eye of a mammal.

10. The use of claim 9, wherein the wound is in the corneal epithelium, stroma and/or endothelium.

11. The use of claim 10, wherein the corneal wound is a corneal ulcer.

12. The use according to anyone of claims 1 to 11, wherein the wound and/or wound healing disorder is caused by infectious diseases, moistening disorders, localized or generalized immunological diseases, associated dermal disorders, ocular manifestation of systemic disease, physical injuries, corneal dystrophies and degenerations and/or surgical intervention.

13. The use according to claim 12, wherein the wound or wound healing disorder is caused by bacterial infection, viral infection, fungal infection keratokonjunctivitis, pemphigoid, Stevens-Johnson Syndrome, rosazea keratitis, ichthyosis, diabetes, rheumatoid arthritis, Morbus Crohn, Morbus Wegener, Lupus Erythematodes, sclerodermia, Terrien's degeneration, Salzman's degeneration, Fuchs' dystrophy and/or laser surgery.

14. The use according to anyone of claims 1 to 13, wherein the pharmaceutical composition additionally contains a pharmaceutically acceptable carrier and/or diluent.

15. The use of claim 14, wherein the carrier is hyaluronic acid or a contact lens or shield.

16. The use according to anyone of claims 1 to 15, wherein the pharmaceutical composition is applied orally, topically, intravenously and/or parenterally.

17. The use of claim 16, wherein the pharmaceutical composition for topical application is formulated as eye drops, a gel formulation, an ointment or a solution for ocular injection.

## Patentansprüche

1. Verwendung eines aus Gliazelllinien stammenden Wachstumfaktors (GDNF) oder eines funktionell aktiven Derivates oder Teiles davon und/oder eines Agonisten, welcher die funktionelle Aktivität von GDNF ersetzt, und/oder einer Nukleinsäure, die mindestens eine Nukleotidsequenz enthält, welche die primäre Aminosäuresequenz von GDNF oder des funktionell aktiven Derivates oder eines Teiles davon und/oder den Agonisten kodiert, für die Herstellung einer pharmazeutischen Zusammensetzung für epidermale und stromale Wundheilung und/oder für die Behandlung von epidermalen und stromalen Wundheilungsstörungen und/oder Vernarbungsstörungen.

2. Verwendung nach Anspruch 1, wobei die pharmazeutische Zusammensetzung humanes rekombinantes GDNF oder ein funktionell aktives Derivat oder einen Teil davon umfaßt.

3. Verwendung nach Anspruch 1, wobei die pharmazeutische Zusammensetzung eine Nukleinsäure umfaßt, welche mindestens die Nukleotidsequenz enthält, welche das humane Wildtyp-GDNF kodiert.

4. Verwendung nach einem der Ansprüche 1 bis 3, wobei die pharmazeutische Zusammensetzung mindestens ein weiteres Mittel mit einer trophischen Wirkung auf Epithel- und/oder neuronale Zellen enthält.

5. Verwendung nach Anspruch 4, wobei das Mittel aus der Gruppe, bestehend aus TGF-βs, BMPs, GDFs, Activin/Inhibin, Neutrophinen, EGF, HB-EGF, TGF-α, FGFs, KGF, HGF, IGFs, PDGFs, Fibronectin und Metaboliten davon, ausgewählt ist.

6. Verwendung nach einem der Ansprüche 1 bis 5, wobei die pharmazeutische Zusammensetzung mindestens ein weiteres Mittel mit einer entzündungshemmenden Wirkung enthält.

7. Verwendung nach Anspruch 6, wobei das entzündungshemmende Mittel aus der Gruppe, bestehend aus Cortisonanalogen, Hemmstoffen des NF-κB-Weges. Hemmstoffen des Arachidonsäureweges und Antikörpern gegen Chemokinen, ausgewählt ist.

8. Verwendung nach einem der Ansprüche 1 bis 7, wobei die pharmazeutische Zusammensetzung Zellen umfaßt, welche GDNF oder das funktionell aktive Derivat oder einen Teil davon und/oder den Agonisten davon erzeugen.

9. Verwendung nach einem der Ansprüche 1 bis 8, wobei die Wunde im vorderen Auge eines Säugers ist.

10. Verwendung nach Anspruch 9, wobei die Wunde im Hornhautepithel, Stroma und/oder Endothel ist.

11. Verwendung nach Anspruch 10, wobei die Hornhautwunde ein Homhautgeschwür ist.

12. Verwendung nach einem der Ansprüche 1 bis 11, wobei die Wunde und/oder die Wundheilungsstörung durch Infektionskrankheiten, Benetzungsstörungen, lokalisierte oder generalisierte immunologische Krankheiten, assoziierte Hautstörungen, sich am Auge manifestierende systemische Krankheiten, physische Verletzungen, Horndystrophien und degenerative und/oder chirurgische Eingriffe verursacht wird.

13. Verwendung nach Anspruch 12, wobei die Wunde oder die Wundheilungsstörung durch bakterielle Infektion, virale Infektion, Pilzinfektion, Keratokonjunktivitis, Pemphigoid, dem Stevens-Johnson-Syndrom, Rosazea-Keratitis, Ichthyosis, Diabetes, rheumatische Arthritis, Morbus Crohn, Morbus Wegener, Lupus Erythematodes, Sklerodermia, Terry-Syndrom, Salzman-Degeneration, Fuchs-Dystrophy und/oder Laserchirurgie verursacht wird.

14. Verwendung nach einem der Ansprüche 1 bis 13, wobei die pharmazeutische Zusammensetzung zusätzlich einen pharmazeutisch verträglichen Träger und/oder Verdünnungsmittel enthält.

15. Verwendung nach Anspruch 14, wobei der Träger Hyaluronsäure oder eine Kontaktlinse oder eine Abschirmung bzw. Schutzglas ist.

16. Verwendung nach einem der Ansprüche 1 bis 15, wobei die pharmazeutische Zusammensetzung oral, topisch, intravenös und/oder parenteral verabreicht wird.

17. Verwendung nach Anspruch 16, wobei die pharmazeutische Zusammensetzung für die topische Anwendung als Augentropfen, Gelformulierung, Salbe oder eine Lösung für Augeninjektion formuliert ist.

## Revendications

1. Utilisation d'un facteur de croissance dérivé d'une lignée de cellules gliales (GDNF) ou d'un de ses dérivés fonctionnellement actifs ou d'une de ses parties fonctionnellement actives et/ou d'un agoniste assurant la substitution de l'activité fonctionnelle du GDNF, et/ou d'un acide nucléique contenant au moins une séquence de nucléotides codant pour la séquence d'aminoacides primaire du GDNF ou de son dérivé fonctionnellement actif ou de sa partie fonctionnellement active et/ou de l'agoniste pour la production d'une composition pharmaceutique destinée à la guérison de plaies épidermiques et stromales et/ou au traitement de troubles de la guérison de plaies épidermiques et stromales et/ou de troubles de la cicatrisation.

2. Utilisation suivant la revendication 1, dans laquelle la composition pharmaceutique comprend du GDNF humain recombinant ou un de ses dérivés fonctionnellement actifs ou une de ses parties fonctionnellement actives.

3. Utilisation suivant la revendication 1, dans laquelle la composition pharmaceutique comprend un acide nucléique contenant au moins la séquence de nucléotides codant pour le GDNF humain de type sauvage.

4. Utilisation suivant l'une quelconque des revendications 1 à 3, dans laquelle la composition pharmaceutique contient au moins un agent supplémentaire ayant un effet trophique sur les cellules épithéliales et/ou cellules neuronales.

5. Utilisation suivant la revendication 4, dans laquelle l'agent est choisi dans le groupe consistant en les TGF-β, les BMP, les GDF, l'activine/inhibine, des neurotrophines, l'EGF, le HB-EGF, le TGF-α, des FGF, le KGF, le HGF, des IGF, des PDGF, la fibronectine et leurs métabolites.

6. Utilisation suivant l'une quelconque des revendications 1 à 5, dans laquelle la composition pharmaceutique contient au moins un agent supplémentaire ayant un effet anti-inflammatoire.

7. Utilisation suivant la revendication 6, dans laquelle l'agent anti-inflammatoire est choisi dans le groupe consistant en des analogues de cortisone, des inhibiteurs de la voie de NF-κB, des inhibiteurs de la voie de l'acide arachidonique et des anticorps contre des chimioquines.

8. Utilisation suivant 'l'une quelconque des revendications 1 à 7, dans laquelle la composition pharmaceutique comprend des cellules qui produisent du GDNF ou son dérivé fonctionnellement actif ou sa partie fonctionnellement active et/ou son agoniste.

9. Utilisation suivant l'une quelconque des revendications 1 à 8, dans laquelle la plaie est située à la partie antérieure de l'oeil d'un mammifère.

10. Utilisation suivant la revendication 9, dans laquelle la plaie est située dans l'épithélium, le stroma et/ou l'endothélium de la cornée.

11. Utilisation suivant la revendication 10, dans laquelle la plaie de la cornée est un ulcère de cornée.

12. Utilisation suivant l'une quelconque des revendications 1 à 11, dans laquelle la plaie et/ou le trouble de guérison d'une plaie est provoqué par des maladies infectieuses, des troubles de l'hydratation, des maladies immunologiques localisées ou généralisées, des troubles dermiques associés, une manifestation oculaire d'une maladie systémique, des lésions physiques, des dystrophies et dégénérescences et/ou une intervention chirurgicale de la cornée.

13. Utilisation suivant la revendication 12, dans laquelle la plaie ou le trouble de guérison d'une plaie est provoquée par une infection bactérienne, une infection virale, une infection fongique, une kérato-conjonctivite, un pemphigoïde, le syndrome de Stenvens-Johnson, la kératite rosacée, l'ichthyose, le diabète, l'arthrite rhumatoïde, la maladie de Crohn, le syndrome de Wegener, le lupus érythémateux, la sclérodermie, la dégénérescence de Terrien, la dégénérescence de Salzman, la dystrophie de Fuchs et/ou une intervention chirurgicale par laser.

14. Utilisation suivant l'une quelconque des revendications 1 à 13, dans laquelle la composition pharmaceutique contient en outre un support et/ou diluant pharmaceutiquement acceptable.

15. Utilisation suivant la revendication 14, dans laquelle le support est l'acide hyaluronique ou une lentille de contact ou une protection.

16. Utilisation suivant l'une quelconque des revendications 1 à 15, dans laquelle la composition pharmaceutique est appliquée par voie orale topique, intraveineuse et/ou parentérale.

17. Utilisation suivant la revendication 16, dans laquelle la composition pharmaceutique pour application topique est formulée sous forme de gouttes ophtalmiques, d'une formulation de gel, d'une pommade ou d'une solution pour injection oculaire.
